# EUROPEAN PATENT APPLICATION

(11) **EP 4 421 082 A1**
(43) Date of publication of application: **28.08.2024**
(21) Application number: 22882338.1
(22) Date of filing: 30.06.2022
(51) Int. Cl.: C07K 5/117, A61K 38/07, A61P 25/28, A61P 25/16

(54) **POLYPEPTIDE AND APPLICATION THEREOF AS CCK RECEPTOR AGONIST/ANTAGONIST**

(30) Priority: 20.10.2021 CN 202111222161
(71) Applicant: Centre for Regenerative Medicine and Health, Hong Kong Institute of Science & Innovation, Chinese Academy of Sciences Limited, N.T., Hong Kong (HK); City University of Hong Kong, Kowloon, Hong Kong (HK)
(72) Inventor: TORTORELLA, Mickey Daniel, Hong Kong Science Park, Pak Shek Kok N.T., Hong Kong (CN); HE, Jufang, Hong Kong Science Park, Pak Shek Kok N.T., Hong Kong (CN)
(74) Representative: Patentanwälte Ruff, Wilhelm, Beier, Dauster & Partner mbB
(86) International application number: PCT/CN2022/102744
(87) International publication number: WO 2023/065716

(57) **Abstract**

The present invention relates to the field of chemical and pharmaceutical technologies, in particular to a polypeptide and its use as a CCK receptor agonist/antagonist. Experiments show that the polypeptide provided by the present invention has a high agonistic/antagonistic activity on CCK receptors. Compared with the polypeptide CCK4, the polypeptide according to the present invention has a longer half-life and duration of efficacy in vivo. Experiments show that the polypeptide according to the present invention produces a significant improvement on the spatial memory deficiency of senile mice, memory-deficient senile mice, and Alzheimer's mice, and thus has a great application potential.

## Description

### TECHNICAL FIELD

The present invention relates to the field of chemical and pharmaceutical technologies, in particular to a polypeptide and its use as a CCK receptor agonist/antagonist.

### BACKGROUND TECHNOLOGY

Cholecystokinin (CCK) is a humoral regulator synthesized and secreted by duodenal and jejunal cells and is a polypeptide consisting of 33 amino acids. It is widely distributed in the brain, especially in the cortex, striatum, hippocampus, cerebral pretectum, diaphragm, and hypothalamus, and is also secreted peripherally through the small intestine, playing an important role as a neurotransmitter or neuromodulator. Its main physiological effects are to contract the gallbladder, stimulate the secretion of pancreatic enzymes, increase the release of insulin, increase the secretion of hepatic bile, delay gastric emptying, stimulate mucus gland secretion, enhance small intestinal peristalsis and inhibit the absorption of potassium, sodium, chlorine and fluid by the jejunum and ileum. In some cases, it can have an effect on arterial pressure and can affect the immune system. In some central neurons, CCK coexists with dopamine. It plays an important role in the mechanism involving acetylcholine, γ-aminobutyric acid, serotonin, opiates, growth hormone suppression, substance P and ion channels. Taking CCK can cause physiological changes, ptosis, hypothermia, hyperglycemia and stubbornness, and some behavioral changes, lack of exercise, decreased aggressiveness, lack of pain, impact on learning knowledge, changes in sexual behavior and satiety when eating.

For CCK to play its biological role, it should firstly bind to CCK receptors present on the surface of its target cells. CCK receptors belong to G protein-coupled receptors and are widely distributed in vivo. CCK receptors include two subtypes based on their affinity for endogenous ligands, namely CCK-A and CCK-B receptors. Two subtypes have been identified in both peripheral and central nervous systems. Experiments have shown that CCK receptor agonists and antagonists can be used to treat diet, obesity, gallbladder cancer, pancreatic cancer, epilepsy, depression, and digestive disorders caused by excess gastric acid. However, the treatment of amnesia and dementia with CCK receptor agonists has not been reported.

At present, the existing peptide CCK receptor agonists and antagonists have a short half-life and cannot have a long-term effect in vivo by oral administration or injection, so the pharmaceutical significance is not much. Therefore, the development of new CCK receptor agonists will have great application significance for the treatment of related diseases.

### SUMMARY OF THE INVENTION

In view of this, the present invention provides a polypeptide and its use as a CCK receptor agonist/antagonist. The polypeptide has a high agonistic/antagonistic activity on CCK receptors. Compared with CCK4, the polypeptide according to the present invention has a longer half-life and duration of efficacy in vivo, and produces an improvement on the spatial memory deficiency of memory-deficient senile mice, and Alzheimer's mice.

In order to achieve the above inventive objective, the present invention provides the following technical solutions:
The present invention provides a polypeptide having a structure shown in Formula I or its stereoisomer, prodrug, pharmaceutically acceptable solvate or salt: 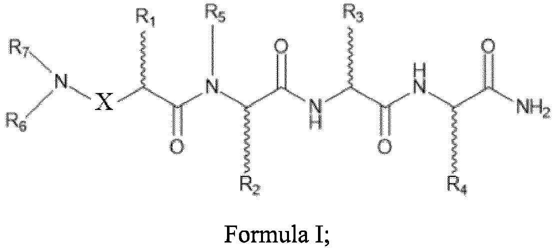 wherein: X is an amide bond or a single bond; 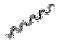 is 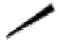 or 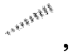 so that the amino acid at the corresponding position is a D-type or L-type amino acid;
R₁, R₂, R₃ and R₄ are independently selected from the group consisting of the following structures: 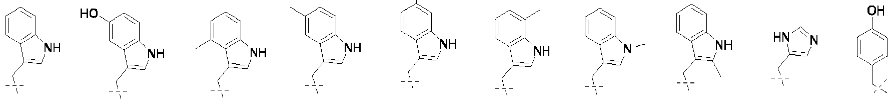 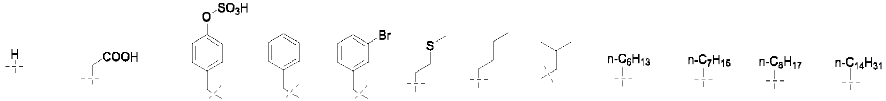 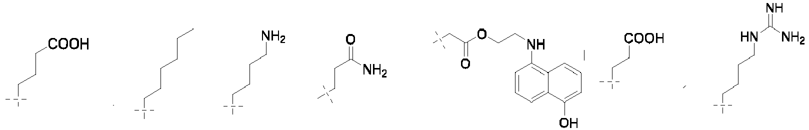 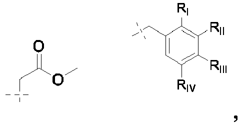 and R_{I}, R_{II}, R_{III} and R_{IV} are independently selected from the group consisting of halogen, nitro, C₁-C₄ alkyl, azido N₃ and cyano;
R₅ and R₆ are independently selected from the group consisting of H, and substituted or unsubstituted C₁-C₄ alkyl;
R₇ is selected from the group consisting of the following structures:
Biotin, AC, Fmoc, Cbz, PEG100, PEG200, PEG300, PEG400, PEG600, PEG800, PEG1000, PEG1500, PEG2000,

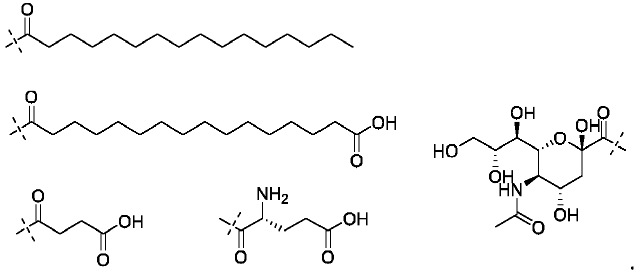

Preferably, R₁ is selected from the group consisting of the following structures:

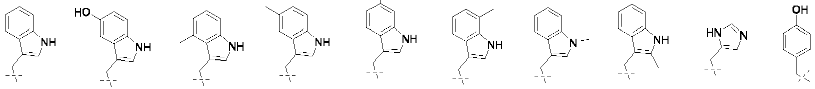

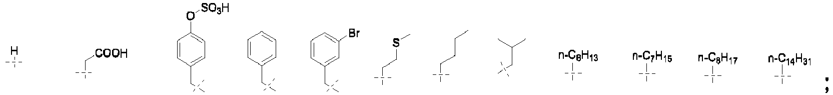

R₂ is selected from the group consisting of the following structures:

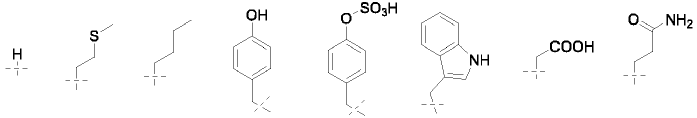

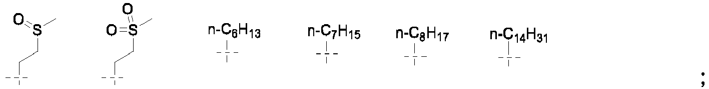

R₃ is selected from the group consisting of the following structures:

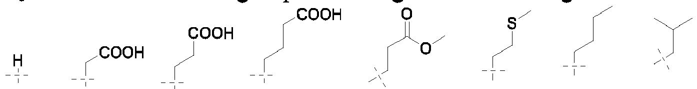

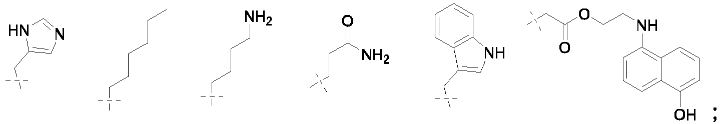

R₄ is selected from the group consisting of the following structures:

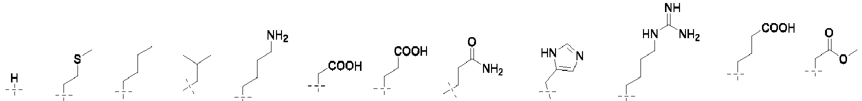

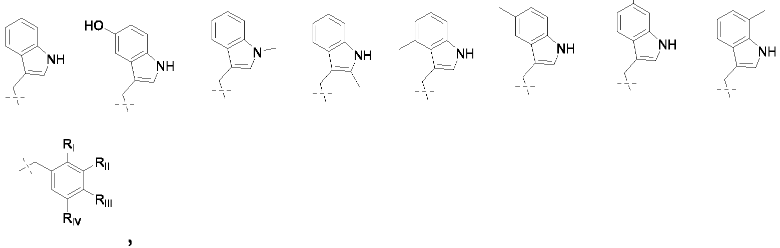

R_{I}, R_{II}, R_{III} and R_{IV} are independently selected from the group consisting of halogen, nitro, C₁-C₄ alkyl, azido and cyano.

Preferably, the polypeptide has a structure shown in Formula II:

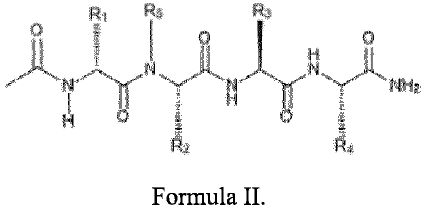

Preferably, in the polypeptide shown in Formula II, R₁ is selected from the group consisting of the following structures:

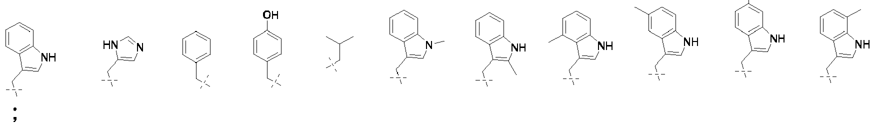

R₂ is selected from the group consisting of the following structures:

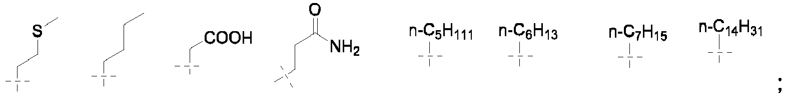

R₃ is selected from the group consisting of the following structures:

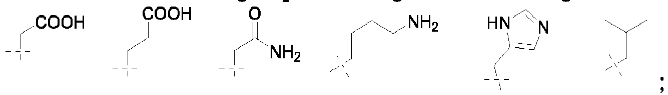

R₄ is selected from the group consisting of the following structures:

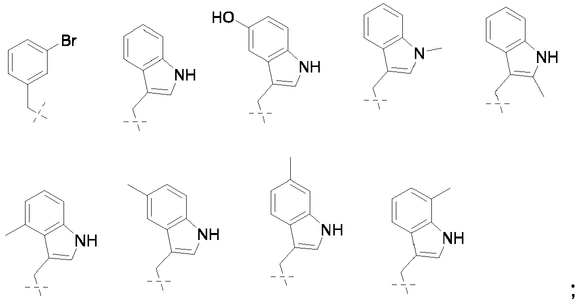

R₅ is selected from the group consisting of H and CH₃.

Preferably, the polypeptide has a structure shown in Formula III or IV: 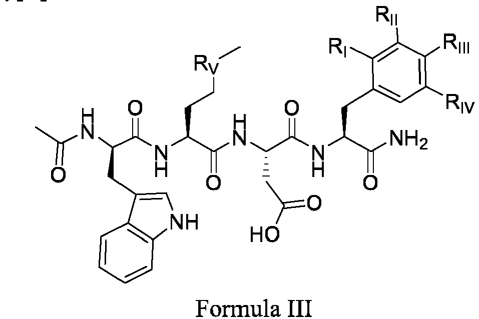 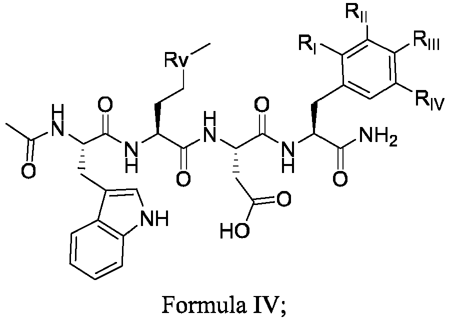 wherein R_{I}, R_{II}, R_{III} and R_{IV} are independently selected from the group consisting of halogen, nitro, C₁-C₄ alkyl, azido N₃ and cyano, and R_{V} is a carbon or sulfur atom.

Preferably, in the polypeptide shown in Formula III, R_{I}, R_{II}, R_{III} and R_{IV} are independently selected from the group consisting of H, F, Cl, Br, I, CN, N₃, Me or NO₂.

In particular, the polypeptide provided by the present invention has any of the structures in Table 1.

Preferably, the polypeptide has a structure shown in Compounds 1-4: (1) Ac-(D-Trp)-Met-Asp-Phe(3-Br)-NH₂

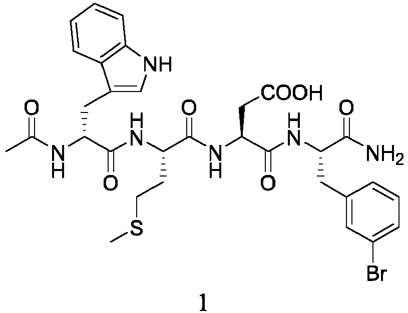

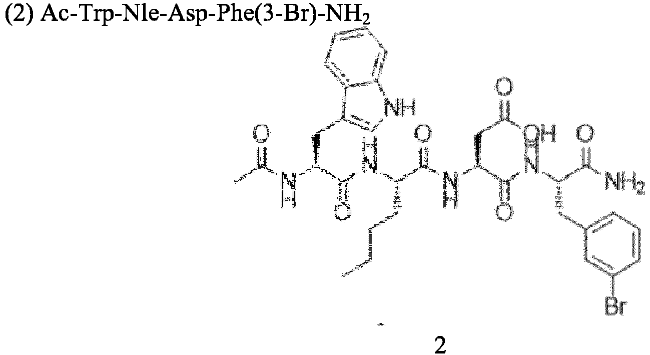

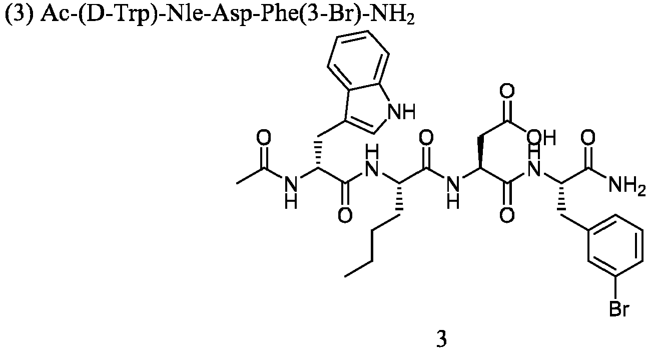

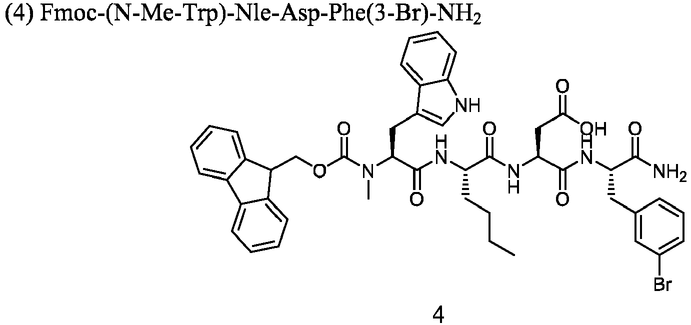

The polypeptide of the present invention is prepared by condensation reaction using amino acids and modified amino acids as raw materials. The present invention has no special limitation on synthesis methods, and solid phase or liquid phase synthesis can be used. The present invention has no special limitation on particular steps of condensation reaction and reagents used, which are commonly used or well-known in the art.

In another respect, the present invention relates to a pharmaceutical composition, comprising the polypeptide described in the present invention, a pharmaceutically acceptable salt, stereoisomer or prodrug molecule thereof, and pharmaceutically acceptable excipients.

"Pharmaceutically acceptable excipients" may include pharmaceutically acceptable carriers, diluents, preservatives, solubilizers, stabilizers, disintegrants, adhesives, lubricants, wetting agents, emulsifiers, sweeteners, colorants, flavoring agents, salts, buffers, coating agents and antioxidants. Those skilled in the art know suitable excipients and techniques for formulating pharmaceutical compositions.

In vitro CCK-B receptor agonism and antagonism experiments on the compounds are performed in the present invention. The experiments have shown that the above compounds have a good agonistic or antagonistic effect on CCK-B receptors, and can be used for the treatment and prevention of diseases that require stimulation of cholecystokinin receptors by complete or partial agonism or antagonism, such as obesity, depression, amnesia, or senile dementia. Thus, the present invention also relates to the use of the polypeptides of any structure shown in Formula I, II, III or IV as a CCK receptor agonist or antagonist, or in the preparation of a medicament for treating or preventing CCK receptor-related diseases.

In the present invention, the CCK receptor-related diseases include at least one of amnesia, dementia, epilepsy, depression, obesity, gallbladder cancer, pancreatic cancer, and diseases of the digestive system.

Among them, amnesia includes anterograde amnesia and retrograde amnesia, and dementia includes Alzheimer's disease (senile dementia), frontotemporal dementia, Parkinson's disease, Prion disease (Creutzfeldt-Jakob disease), Lewy body dementia, Huntington's disease, etc. "Treating or preventing" amnesia or dementia includes, in particular, but is not limited to, improving or partially improving amnesia, improving the learning process, improving memory ability, preventing or reversing amnesia episodes, improving or preventing amnesia or dementia complications, such as anxiety, depression, irritability, loss of interest, social withdrawal, etc. In a specific embodiment of the present invention, the administration of the polypeptide having a structure of Formula I to a subject may significantly improve the subject's learning ability and improve the subject's memory deficiency, and has a long-term effect.

The expression "effective amount" usually means an amount sufficient to produce a therapeutically desired outcome, where the exact nature of the outcome varies according to the specific condition being treated. The polypeptide of the present invention having a structure of Formula I may be included in a composition, especially a pharmaceutical composition, in an effective amount, i.e., an amount suitable for the treatment or prevention of amnesia or dementia of the subject, especially a mammal.

The subject may be a human or an animal, in particular the subject is a mammal, preferably a human. Therefore, the subject is preferably a human with amnesia or dementia. The effective amount of the polypeptide of the present invention having a structure of Formula I may depend on the species, weight, age and individual conditions of the subject, and may be determined by standard methods such as laboratory animals.

Those skilled in the art may determine whether the subject needs to be treated for amnesia or dementia according to the present invention based on predisposing factors. Predisposing factors for amnesia or dementia can include, but are not limited to, any type of injury or trauma to central nervous system (CNS), including degenerative and non-degenerative diseases of central nervous system, the degenerative diseases such as Alzheimer's disease, frontotemporal dementia, Prion disease (Creutzfeldt-Jakob disease), Lewy body dementia, Parkinson's disease, Huntington's disease, etc., and the non-degenerative diseases such as vascular dementia, space-occupying lesions (tumors, chronic subdural hematomas, chronic brain abscesses), infections (meningoencephalitis, neurosyphilis, AIDS dementia, Prion disease), traumatic dementia of the brain, normal intracranial pressure hydrocephalus, endocrine metabolism disorders, poisoning, hypoxia and paraneoplastic syndromes, etc..

The polypeptides according to the present invention having a structure of Formula I may be administered orally, by injection, rectally, locally, parenterally, percutaneously, or by inhalation to the subject. In the embodiment in which the subject is a mouse, the polypeptide having a structure of Formula I is administered to the subject by injection. The term injection includes intraperitoneal, intravenous, intramuscular, subcutaneous, and intradermal administration.

Compared with the prior art, the polypeptide of the present invention has a longer in vivo half-life, and a longer duration of efficacy. Experiments show that the polypeptide produces a significant improvement on memory of senile and memory-deficient mouse models, and thus has a great application potential.

### BRIEF DESCRIPTION OF DRAWINGS

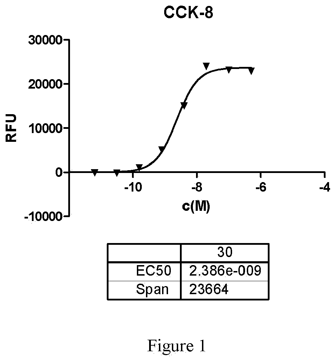

Figure 1 is a graph showing the effect of CCK8 on CHO-CCK cells.

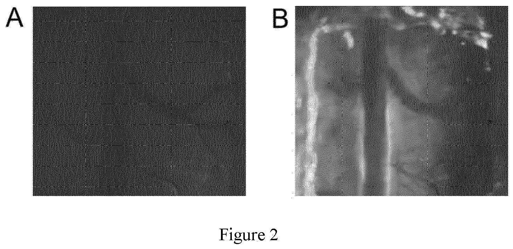

Figure 2 shows the fluorescence signal observation results of the posterior visual cortex in different treatment groups. A is the result of blank control group without injection of compound HT-177, and B is the result of intraperitoneal injection of compound HT-177.

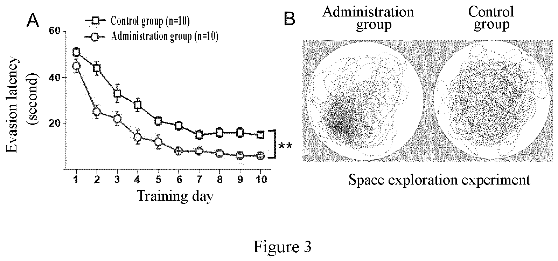

Figure 3 shows that intraperitoneal injection of CCK4 improves the behavioral performance of senile mice in the Morris water maze test. (A) Daily learning performance of mice in the administration group and control group in the positioning navigation experiment; (B) Track diagrams of mice in the administration group and control group in the space exploration experiment.

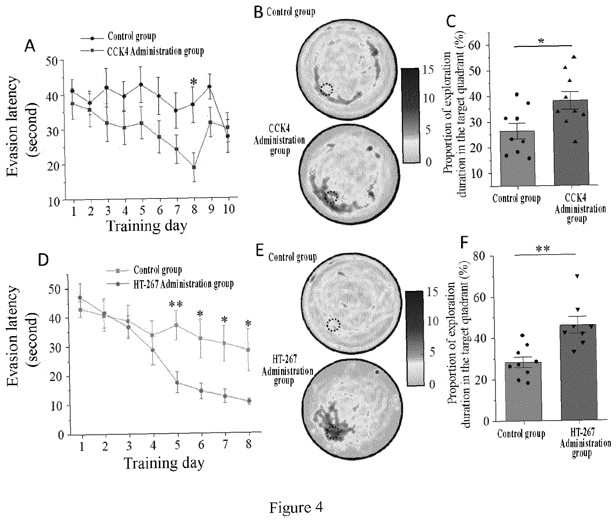

Figure 4 shows that intraperitoneal injection of CCK4 and compound HT-267 improves the behavioral performance of Alzheimer's mice in the Morris water maze test. (A) Daily learning performance of mice in the CCK4 administration group and control group in the positioning navigation experiment; (B) Track heat map of mice in the CCK4 administration group and control group in the space exploration experiment, with dotted circles indicating the location of the original platform; (C) The proportion of the target quadrant exploration duration of mice in the CCK4 administration group and control group in the space exploration experiment; (D) Daily learning performance of mice in the HT-267 administration group and control group in the positioning navigation experiment; (E) Track heat map of mice in the HT-267 administration group and control group in the space exploration experiment, with dotted circles indicating the location of the original platform; (F) The proportion of the target quadrant exploration duration of mice in the HT-267 administration group and control group in the space exploration experiment.

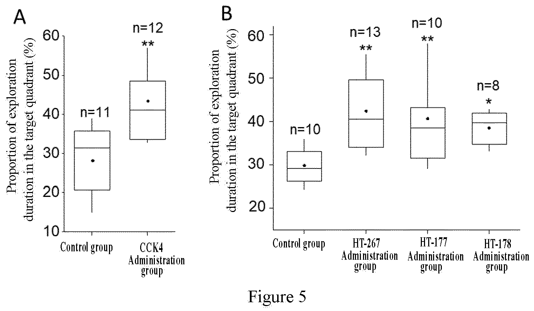

Figure 5 shows that intraperitoneal injection of CCK4 and compounds HT-267, HT-177 and HT-178 improves the behavioral performance of memory-deficient mice in the Morris water maze test. (A) The proportion of the target quadrant exploration duration of mice in the CCK4 administration group and control group in the space exploration experiment; (B) The proportion of the target quadrant exploration duration of mice in the compounds HT-267, HT-177 and HT-178 administration group and control group in the space exploration experiment.

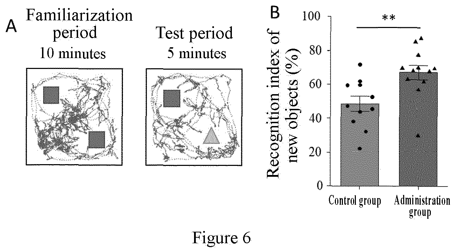

Figure 6 shows that intraperitoneal injection of compound HT-267 improves the behavioral performance of Alzheimer's mice in the new object recognition experiment. (A) Exemplary tracks of mice during the familiarization and test periods, with blue squares representing old objects and yellow triangles representing new objects; (B) Recognition index of new objects by mice in the administration group and control group.

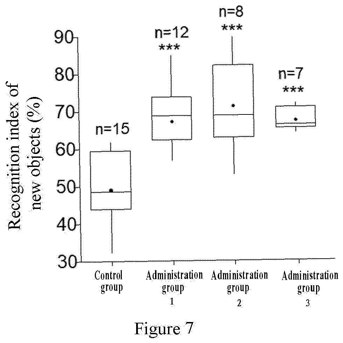

Figure 7 shows the recognition index of new objects by mice in the administration group and control group in the new object recognition experiment. Intraperitoneal injection of compounds HT-267 (administration group 1), HT-177 (administration group 2), and HT-178 (administration group 3) improves the behavioral performance of memory-deficient mice in the new object recognition experiment.

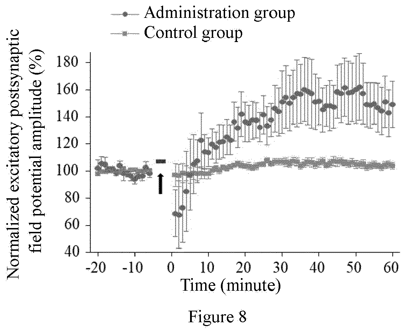

Figure 8 shows the long-term potentiation effect of compound HT-267 (administration group) on the cortex of senile mice. The left side of time point 0 is the basal field potential level before administration, and the right side of time point 0 is the field potential record after administration.

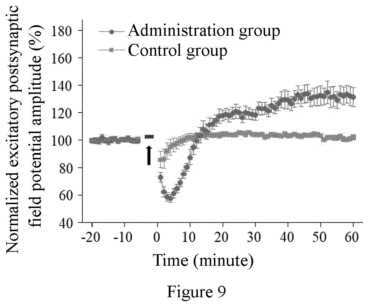

Figure 9 shows the long-term potentiation effect of compound HT-267 (administration group) on the cortex of Alzheimer's mice. The left side of time point 0 is the basal field potential level before administration, and the right side of time point 0 is the field potential record after administration.

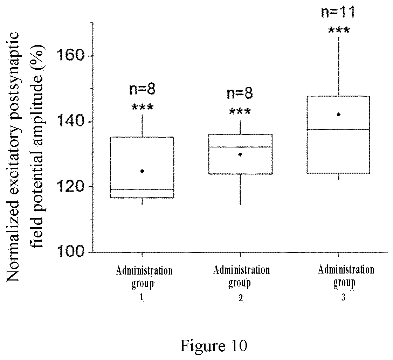

Figure 10 shows the long-term potentiation effect of compounds HT-267 (administration group 1), compound HT-177 (administration group 2), and compound HT-178 (administration group 3) on the cortex of memory deficient mice. 100% is the basal field potential level before administration after normalization, and the field potential levels after administration are shown.

### DETAILED DESCRIPTION

The present invention provides a polypeptide and its use as a CCK receptor agonist/antagonist. Those skilled in the art may refer to the disclosure of the present application to appropriately improve the process parameters. In particular, all similar substitutions and modifications are obvious to those skilled in the art, and they are considered to be included in the present invention. The methods and uses of the present invention have been described by preferable embodiments, and those skilled in the art can obviously modify or appropriately change and combine the methods and uses herein without departing from the disclosure, spirit and scope of the present invention to realize and apply the present invention.

The materials used in the present invention are all commercially available products and can be purchased in the market.

The present invention is further described below in conjunction with the Examples:
Example 1: The relationship between the structure of the polypeptide compound of the present invention and the agonistic activity of the CCK receptor

Using amino acids and modified amino acids as raw materials, polypeptide analogue compounds are generated by condensation reaction, and the activity of polypeptides is determined according to the following process, and the structure and activity are shown in Table 1.
(1) Reagent and material:
   Cell line: CHO-CCK model cells
   Detection reagent: Fluo-8 No Wash Calcium Assay Kit (AAT Bioquest, #36316)
   Positive control: CCK8
(2) Test:
   To detect changes in intracellular calcium ion concentration after the compound acts on model cells containing CCK-B receptors (CHO-CCK).
(3) Method of preparing the compound:
   1. The compound was formulated to a 10 mM mother liquor with DMSO, and then diluted into a series of 9 concentrations with a 3-fold gradient to form a 1000× stock solution.

### 2.1 Agonist

The compound stock solution was subjected to 200-fold dilution with HHBS buffer to form a 5× working solution.

### 2.2 Antagonist

CCK8 was firstly added to the HHBS buffer to a final concentration of 50 nM, and then the compound was diluted 200-fold with the obtained HHBS buffer into a 5× working solution.

### (4) Test scheme:

1. Plate CHO-CCK cells in a 96-well plate with media containing 1% FBS, 30,000 cells/well/100 µL, and incubate overnight at 37°C.
2. Prepare 1× Fluo-8 analysis buffer: mix 9 mL of HHBS, 1 mL of 10× Pluronic F127 Plus and 20 µL of Fluo-8 thoroughly for later use.
3. Add Fluo-8 dye working solution to the cell plate, 100 µL/well. First incubate in a 37°C incubator for 30 min, and then take it out and incubate at room temperature in the dark for 30 min.
4. In the process of incubating cells, the compounds to be detected were prepared with HHBS.
5. Add the compounds to the 96-well CHO-CCK cell plate line by line, 50 µL/well, and then immediately put it into the microplate reader to detect the dynamic fluorescence signal change of Ex/Em=490 nm/520 nm, line by line.

### (5) Test result and analysis method:

1. The effect of CCK8 on CHO-CCK cells is shown in Figure 1.

The results showed that CCK8, as a positive control, was considered to have the strongest agonistic activity, and its high-concentration effect was the saturated state of cells. The concentration effect curve of CCK8 was analyzed, and the concentration where its agonistic effect reaches 90% saturation, that is, EC₉₀ was calculated as about 10 nM, so the concentration of CCK8 in the antagonist screening method was set to 10 nM. The percentage of signal of cell group of the compound and 10 nM CCK8 acting simultaneously accounting for that of cell group of 10 nM CCK8 alone was calculated when analyzing the results.

The agonistic effect of CCK8 at 20 nM reached substantial saturation, so the fluorescence intensity corresponding to this concentration was used as a 100% control in the analysis of agonist screening results. The percentage of signal of cell group of the compound alone accounting for that of cell group of 20 nM CCK8 alone was calculated when analyzing the results.

The agonism/antagonism EC₅₀ of the compound can be obtained by plotting the fitting curve with the agonism/antagonism percentage as Y-axis and the concentration of the compound as X-axis.

**Table 1: Structures and activities of polypeptides**

| **No.** | **Sequence** | **Structure** | **EC50 (M) agonist** | **EC50 (M) antagonis t** | **Agonis tic Activit y (%) at 10 µM** | **%Act-Ave @ 50 nM** | **%Act-Ave @ InM** |
|---|---|---|---|---|---|---|---|
| HT-1 | Phe-Asp-Met-Trp-COOH | 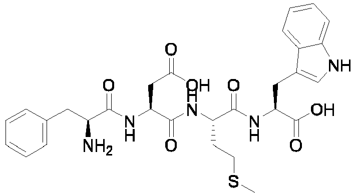 | >10 µM | N.A. | N.A. | N.A. | N.A. |
| HT-2 | Ac-Phe-Asp-Met-Trp-COOH | 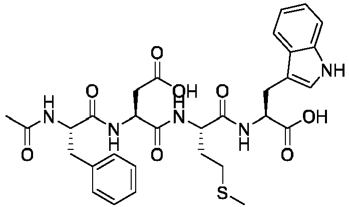 | >10 µM | N.A. | N.A. | N.A. | N.A. |
| HT-3 | Ac-Phe-Asp-Met-Trp-NH2 | 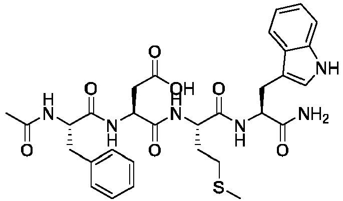 | >10 µM | N.A. | N.A. | N.A. | N.A. |
| HT-4 | Ac-Phe-D-Asp-Met-Trp-NH2 | 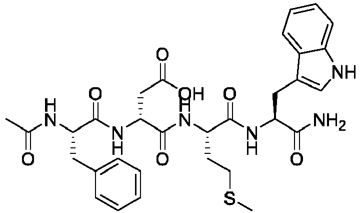 | >10 µM | N.A. | N.A. | N.A. | N.A. |
| HT-5 | Ac-Phe-Asp-D-Met-Trp-NH2 | 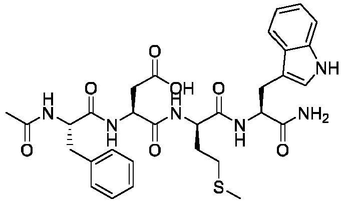 | >10 µM | N.A. | N.A. | N.A. | N.A. |
| HT-6 | Ac-Phe-Asp-Met-D-Trp-NH2 | 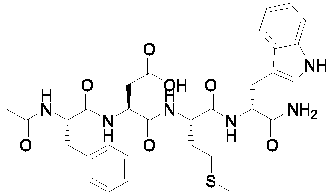 | >10 µM | N.A. | N.A. | N.A. | N.A. |
| HT-7 | D-Phe-D-Asp-D-Met-D-Trp-COOH | 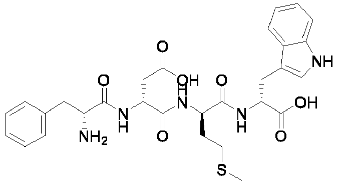 | >10 µM | N.A. | N.A. | N.A. | N.A. |
| HT-8 | Ac-D-Phe-D-Asp-D-Met-D-Trp-NH2 | 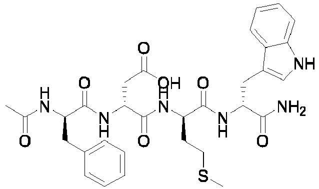 | >10 µM | N.A. | N.A. | N.A. | N.A. |
| HT-9 | Trp-Met-Asp-Phe-NH2 | 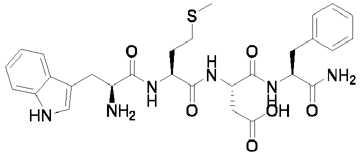 | 1.94E-08 | >50 µM | N.A. | 94.51 | 68.19 |
| HT-10 | D-Trp-Met-Asp-Phe-NH2 | 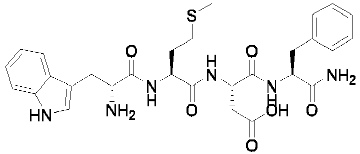 | 2.11E-07 | >50 µM | N.A. | N.A. | N.A. |
| HT-11 | Trp-D-Met-Asp-Phe-NH2 | 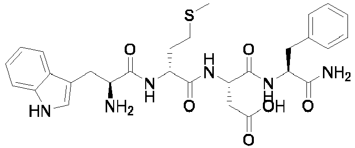 | 2.49E-05 | >50 µM | N.A. | N.A. | N.A. |
| HT-12 | Trp-Met-D-Asp-Phe-NH2 | 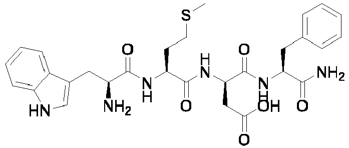 | 6.27E-06 | >50 µM | N.A. | N.A. | N.A. |
| HT-13 | Trp-Met-Asp-D-Phe-NH2 | 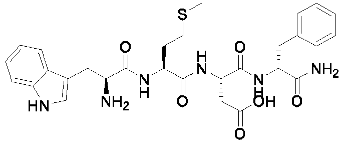 | 4.02E-06 | >50 µM | N.A. | N.A. | N.A. |
| HT-14 | Trp-Met-Asp-Phe-COOH | 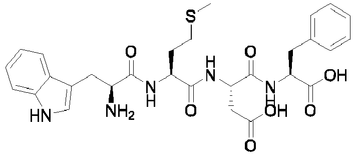 | 6.48E-06 | >50 µM | N.A. | N.A. | N.A. |
| HT-15 | D-Trp-D-Met-D-Asp-D-Phe-NH2 | 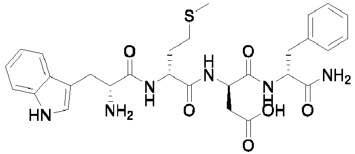 | 1.54E-05 | >50 µM | N.A. | N.A. | N.A. |
| HT-16 | D-Trp-Met(O)-Asp-Phe-NH2 | 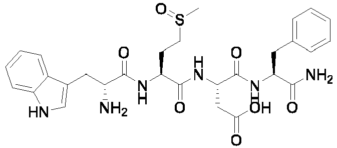 | N.A. | N.A. | 19.11 | N.A. | N.A. |
| HT-17 | D-Trp-Met(O2)-Asp-Phe-NH2 | 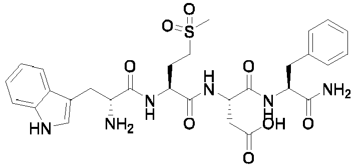 | N.A. | N.A. | 19.12 | N.A. | N.A. |
| HT-18 | D-Trp-Met-Asp(OMe)-Phe-NH2 | 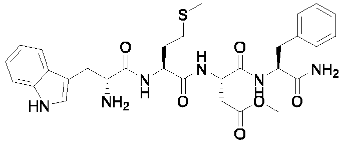 | N.A. | N.A. | 7.59 | N.A. | N.A. |
| HT-19 | D-Trp-Met-Asp(EDAN S)-Phe-NH2 | 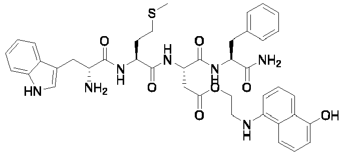 | N.A. | N.A. | -7.97 | N.A. | N.A. |
| HT-20 | D-Trp-Met(O)-Asp(OMe)-Phe-NH2 | 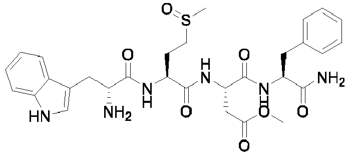 | N.A. | N.A. | -9.73 | N.A. | N.A. |
| HT-21 | D-Trp-Met(O)-Asp(EDAN S)-Phe-NH2 | 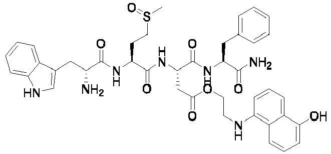 | N.A. | N.A. | -9.61 | N.A. | N.A. |
| HT-22 | D-Trp-Met(O2)-Asp(OMe)-Phe-NH2 | 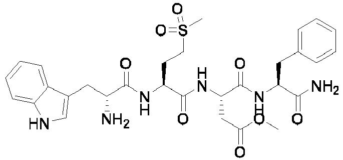 | N.A. | N.A. | 0.98 | N.A. | N.A. |
| HT-23 | D-Trp-Met(O2)-Asp(EDAN S)-Phe-NH2 | 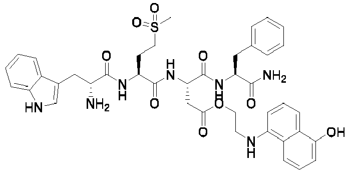 | N.A. | N.A. | -12.85 | N.A. | N.A. |
| HT-24 | D-Trp-Met-Asp-Phe(2-NO2)-NH2 | 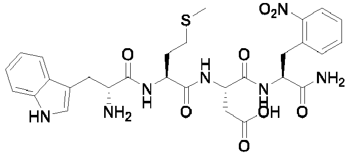 | 2.06E-07 | 6.24E-07 | 50.60 | N.A. | N.A. |
| HT-25 | D-Trp-Met-Asp-Phe(2-F)-NH2 | 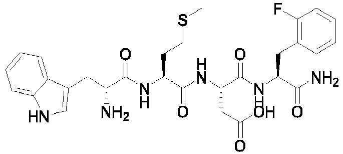 | 2.25E-07 | 6.03E-07 | 89.41 | N.A. | N.A. |
| HT-26 | D-Trp-Met-Asp-Phe(3-C1)-NH2 | 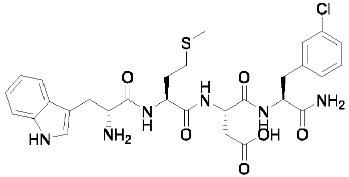 | 7.84E-08 | 6.35E-07 | 85.75 | N.A. | N.A. |
| HT-27 | D-Trp-Met-Asp-Phe(3-F)-NH2 | 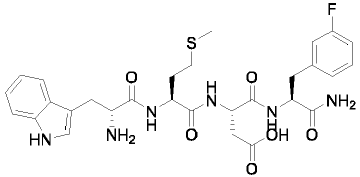 | 1.55E-07 | 1.34E-06 | 79.08 | N.A. | N.A. |
| HT-28 | D-Trp-Met-Asp-Phe(3-I)-NH2 | 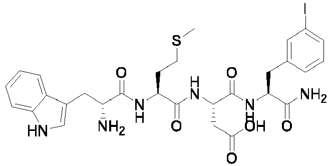 | 1.54E-07 | 1.49E-07 | 49.93 | N.A. | N.A. |
| HT-29 | D-Trp-Met-Asp-Phe(3-Br)-NH2 | 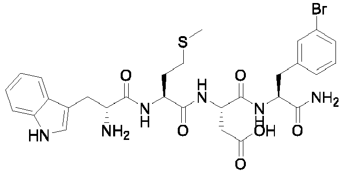 | 8.10E-09 | 1.06E-07 | 85.68 | N.A. | N.A. |
| HT-30 | D-Trp-Met-Asp-Phe(4-Me)-NH2 | 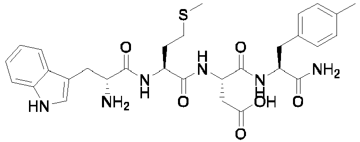 | 9.32E-08 | 6.83E-07 | 88.74 | N.A. | N.A. |
| HT-31 | D-Trp-Met-Asp-Phe(4-N3)-NH2 | 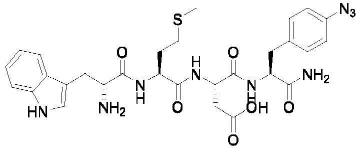 | N.A. | N.A. | 6.07 | N.A. | N.A. |
| HT-32 | D-Trp-Met-Asp-Phe(4-CN)-NH2 | 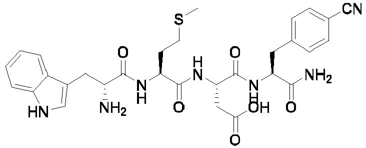 | 1.13E-06 | 2.66E-06 | 57.37 | N.A. | N.A. |
| HT-33 | D-Trp-Met-Asp-Phe(4-NO2)-NH2 | 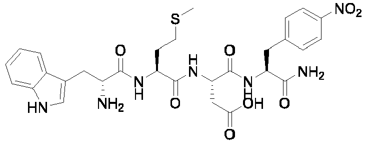 | 2.41E-07 | 1.20E-06 | 71.20 | N.A. | N.A. |
| HT-34 | D-Trp-Met-Asp-Phe(4-Br)-NH2 | 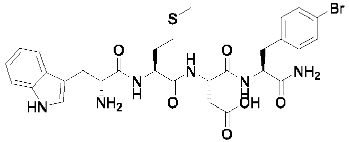 | 3.69E-07 | 2.25E-06 | 98.24 | N.A. | N.A. |
| HT-35 | D-Trp-Met-Asp-Phe(4-C1I)-NH2 | 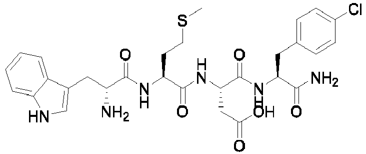 | 1.80E-07 | 7.55E-07 | 109.16 | N.A. | N.A. |
| HT-36 | D-Trp-Met-Asp-Phe(4-F)-NH2 | 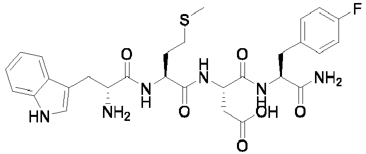 | 8.88E-08 | 8.76E-07 | -3.55 | N.A. | N.A. |
| HT-37 | D-Trp-Met-Asp-Phe(4-I)-NH2 | 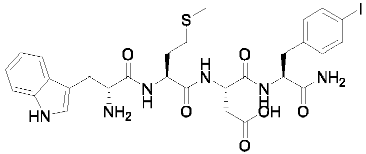 | 8.64E-07 | 1.22E-06 | 52.04 | N.A. | N.A. |
| HT-38 | D-Trp-Met-Asp-Phe(3,5-DiCl)-NH2 | 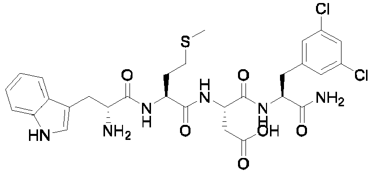 | 4.93E-08 | 9.97E-08 | 122.74 | N.A. | N.A. |
| HT-39 | D-Trp-Met-Asp-Phe(3,5-DiF)-NH2 | 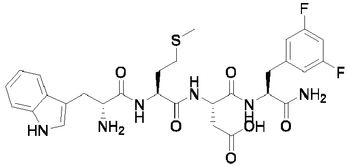 | 4.42E-08 | 6.31E-07 | 119.04 | N.A. | N.A. |
| HT-40 | D-Trp-Met-Asp-Phe(3,4,5-TriF)-NH2 | 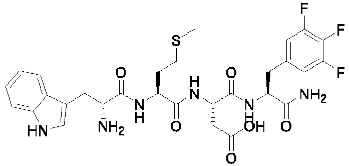 | 7.80E-08 | 2.44E-07 | 98.25 | N.A. | N.A. |
| HT-41 | D-Trp-Met(O)-Asp-Phe(2-NO2)-NH2 | 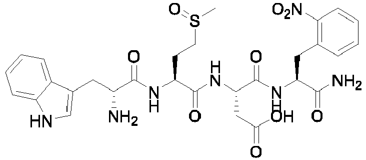 | N.A. | N.A. | -11.17 | N.A. | N.A. |
| HT-42 | D-Trp-Met(O)-Asp-Phe(2-F)-NH2 | 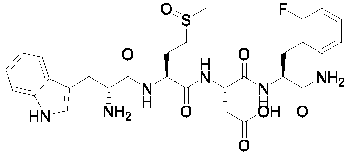 | N.A. | 8.41E-06 | 17.97 | N.A. | N.A. |
| HT-43 | D-Trp-Met(O)-Asp-Phe(3-Cl)-NH2 | 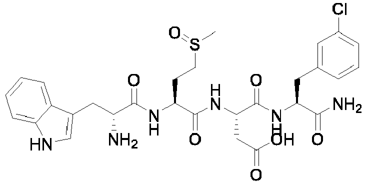 | 3.02E-06 | 8.97E-06 | 62.14 | N.A. | N.A. |
| HT-44 | D-Trp-Met(O)-Asp-Phe(3-F)-NH2 | 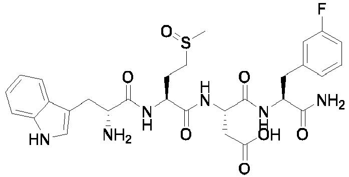 | N.A. | >10µM | 34.63 | N.A. | N.A. |
| HT-45 | D-Trp-Met(O)-Asp-Phe(3-I)-NH2 | 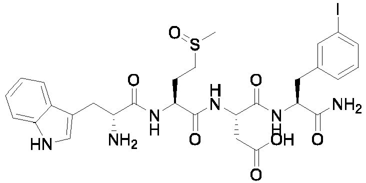 | 4.38E-06 | 3.37E-06 | 58.98 | N.A. | N.A. |
| HT-46 | D-Trp-Met(O)-Asp-Phe(3-Br)-NH2 | 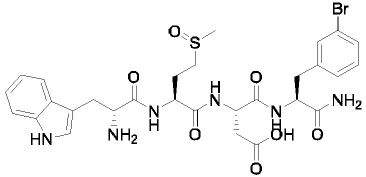 | 3.80E-06 | 6.24E-06 | 53.69 | N.A. | N.A. |
| HT-47 | D-Trp-Met(O)-Asp-Phe(4-Me)-NH2 | 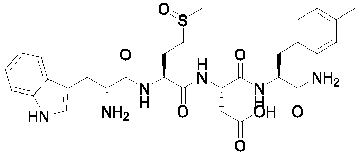 | N.A. | N.A. | 26.99 | N.A. | N.A. |
| HT-48 | D-Trp-Met(O)-Asp-Phe(4-N3)-NH2 | 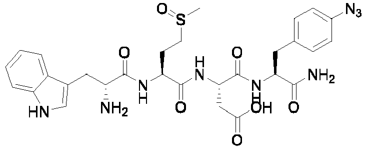 | N.A. | N.A. | 17.23 | N.A. | N.A. |
| HT-49 | D-Trp-Met(O)-Asp-Phe(4-CN)-NH2 | 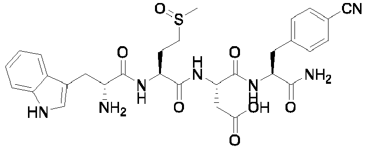 | N.A. | N.A. | 1.03 | N.A. | N.A. |
| HT-50 | D-Trp-Met(O)-Asp-Phe(4-NO2)-NH2 | 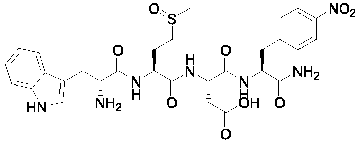 | N.A. | N.A. | 11.85 | N.A. | N.A. |
| HT-51 | D-Trp-Met(O)-Asp-Phe(4-Br)-NH2 | 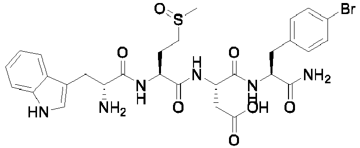 | N.A. | N.A. | 17.18 | N.A. | N.A. |
| HT-52 | D-Trp-Met(O)-Asp-Phe(4-Cl)-NH2 | 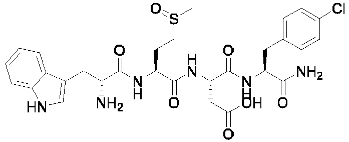 | N.A. | N.A. | 15.71 | N.A. | N.A. |
| HT-53 | D-Trp-Met(O)-Asp-Phe(4-F)-NH2 | 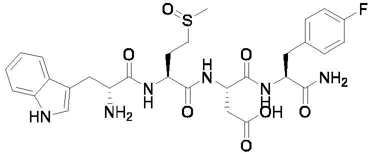 | N.A. | 8.88E-06 | 30.00 | N.A. | N.A. |
| HT-54 | D-Trp-Met(O)-Asp-Phe(4-I)-NH2 | 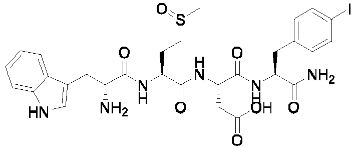 | N.A. | N.A. | 3.14 | N.A. | N.A. |
| HT-55 | D-Trp-Met(O)-Asp-Phe(3,5-DiCl)-NH2 | 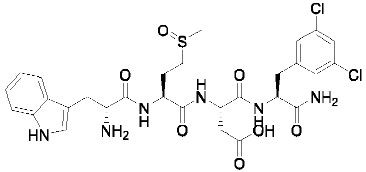 | 1.65E-06 | 1.79E-06 | 62.28 | N.A. | N.A. |
| HT-56 | D-Trp-Met(O)-Asp-Phe(3,5-DiF)-NH2 | 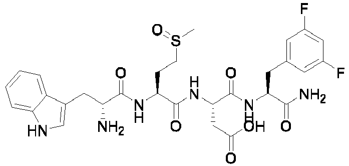 | N.A. | N.A. | 43.98 | N.A. | N.A. |
| HT-57 | D-Trp-Met(O)-Asp-Phe(3,4,5-TriF)-NH2 | 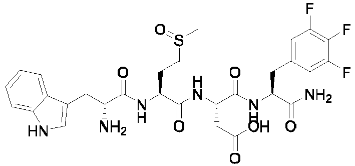 | 6.79E-06 | N.A. | 56.81 | N.A. | N.A. |
| HT-58 | D-Trp-Met(O2)-Asp-Phe(2-NO2)-NH2 | 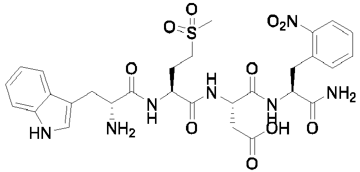 | N.A. | N.A. | 23.63 | N.A. | N.A. |
| HT-59 | D-Trp-Met(O2)-Asp-Phe(2-F)-NH2 | 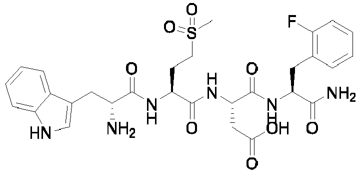 | N.A. | 9.22E-06 | 39.73 | N.A. | N.A. |
| HT-60 | D-Trp-Met(O2)-Asp-Phe(3-Cl)-NH2 | 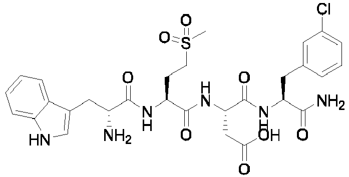 | 9.24E-06 | 6.61E-06 | 50.72 | N.A. | N.A. |
| HT-61 | D-Trp-Met(O2)-Asp-Phe(3-F)-NH2 | 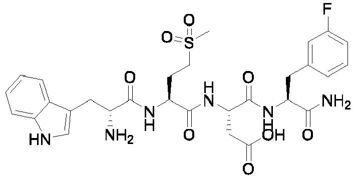 | N.A. | >10µM | 36.66 | N.A. | N.A. |
| HT-62 | D-Trp-Met(O2)-Asp-Phe(3-I)-NH2 | 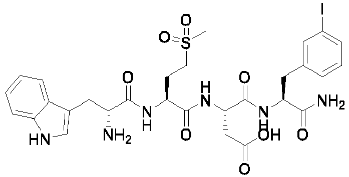 | 4.37E-06 | N.A. | 4.26 | N.A. | N.A. |
| HT-63 | D-Trp-Met(O2)-Asp-Phe(3-Br)-NH2 | 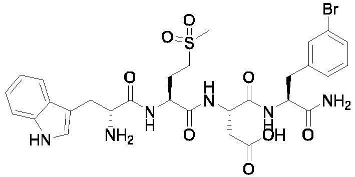 | 6.05E-06 | 7.50E-06 | 48.46 | N.A. | N.A. |
| HT-64 | D-Trp-Met(O2)-Asp-Phe(4-Me)-NH2 | 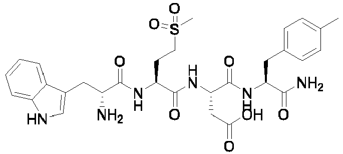 | N.A. | N.A. | 17.43 | N.A. | N.A. |
| HT-65 | D-Trp-Met(O2)-Asp-Phe(4-N3)-NH2 | 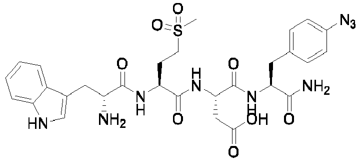 | N.A. | >10µM | 8.35 | N.A. | N.A. |
| HT-66 | D-Trp-Met(O2)-Asp-Phe(4-CN)-NH2 | 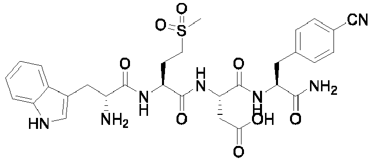 | N.A. | N.A. | 0.33 | N.A. | N.A. |
| HT-67 | D-Trp-Met(O2)-Asp-Phe(4-NO2)-NH2 | 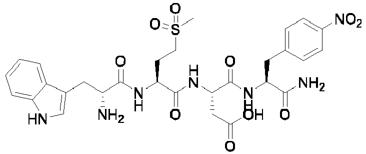 | N.A. | N.A. | -1.49 | N.A. | N.A. |
| HT-68 | D-Trp-Met(O2)-Asp-Phe(4-Br)-NH2 | 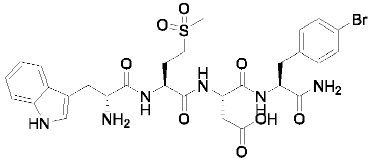 | N.A. | N.A. | 15.61 | N.A. | N.A. |
| HT-69 | D-Trp-Met(O2)-Asp-Phe(4-Cl)-NH2 | 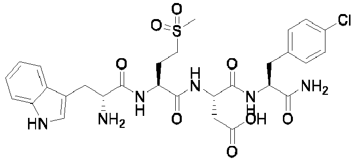 | N.A. | N.A. | 20.02 | N.A. | N.A. |
| HT-70 | D-Trp-Met(O2)-Asp-Phe(4-F)-NH2 | 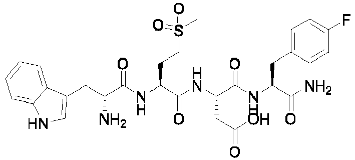 | 1.14E-05 | N.A. | 35.31 | N.A. | N.A. |
| HT-71 | D-Trp-Met(O2)-Asp-Phe(4-I)-NH2 | 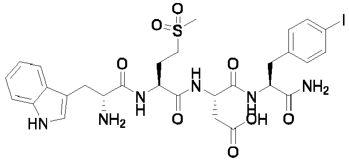 | N.A. | N.A. | 13.13 | N.A. | N.A. |
| HT-72 | D-Trp-Met(O2)-Asp-Phe(3,5-DiCl)-NH2 | 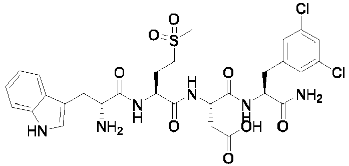 | 1.51E-06 | 2.62E-06 | 82.71 | N.A. | N.A. |
| HT-73 | D-Trp-Met(O2)-Asp-Phe(3,5-DiF)-NH2 | 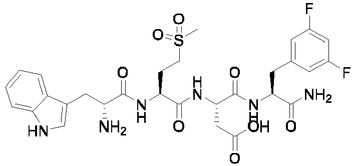 | 1.26E-05 | 8.82E-06 | 55.41 | N.A. | N.A. |
| HT-74 | D-Trp-Met(O2)-Asp-Phe(3,4,5-TriF)-NH2 | 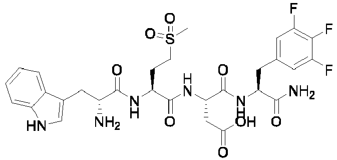 | 1.10E-05 | 8.42E-06 | 55.05 | N.A. | N.A. |
| HT-75 | D-Trp-Met-Asp(Ome)-Phe(2-NO2)-NH2 | 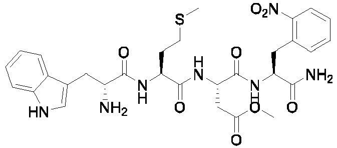 | N.A. | N.A. | -0.20 | N.A. | N.A. |
| HT-76 | D-Trp-Met-Asp(Ome)-Phe(2-F)-NH2 | 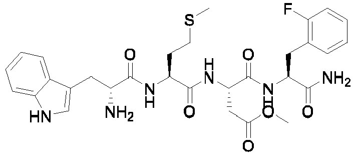 | N.A. | N.A. | 23.09 | N.A. | N.A. |
| HT-77 | D-Trp-Met-Asp(Ome)-Phe(3-Cl)-NH2 | 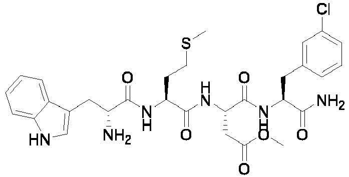 | N.A. | N.A. | 1.83 | N.A. | N.A. |
| HT-78 | D-Trp-Met-Asp(Ome)-Phe(3-F)-NH2 | 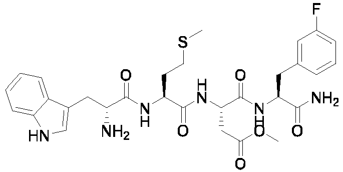 | N.A. | ~10µM | 20.83 | N.A. | N.A. |
| HT-79 | D-Trp-Met-Asp(Ome)-Phe(3-I)-NH2 | 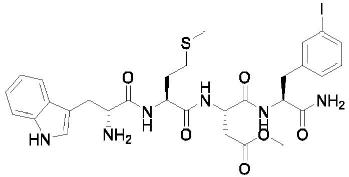 | 2.29E-06 | 1.99E-06 | 38.37 | N.A. | N.A. |
| HT-80 | D-Trp-Met-Asp(Ome)-Phe(3-Br)-NH2 | 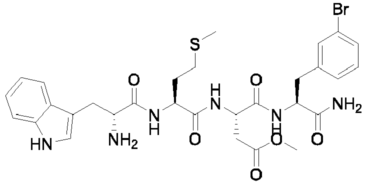 | 5.72E-07 | 8.07E-07 | 57.83 | N.A. | N.A. |
| HT-81 | D-Trp-Met-Asp(Ome)-Phe(4-Me)-NH2 | 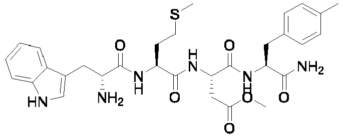 | N.A. | N.A. | 12.27 | N.A. | N.A. |
| HT-82 | D-Trp-Met-Asp(Ome)-Phe(4-N3)-NH2 | 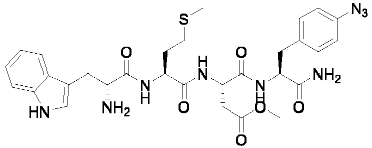 | N.A. | N.A. | 12.78 | N.A. | N.A. |
| HT-83 | D-Trp-Met-Asp(Ome)-Phe(4-CN)-NH2 | 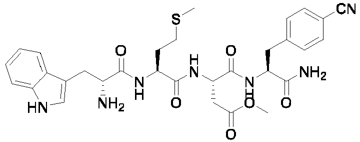 | N.A. | N.A. | 1.91 | N.A. | N.A. |
| HT-84 | D-Trp-Met-Asp(Ome)-Phe(4-NO2)-NH2 | 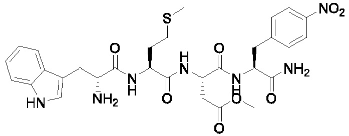 | N.A. | N.A. | 3.24 | N.A. | N.A. |
| HT-85 | D-Trp-Met-Asp(Ome)-Phe(4-Br)-NH2 | 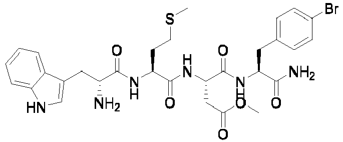 | N.A. | N.A. | 7.32 | N.A. | N.A. |
| HT-86 | D-Trp-Met-Asp(Ome)-Phe(4-Cl)-NH2 | 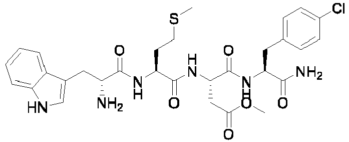 | N.A. | N.A. | -0.94 | N.A. | N.A. |
| HT-87 | D-Trp-Met-Asp(Ome)-Phe(4-F)-NH2 | 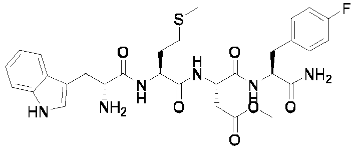 | N.A. | 9.98E-06 | 28.73 | N.A. | N.A. |
| HT-88 | D-Trp-Met-Asp(Ome)-Phe(4-I)-NH2 | 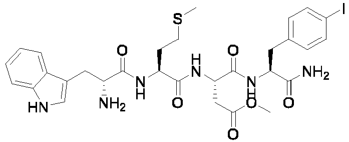 | N.A. | N.A. | -4.64 | N.A. | N.A. |
| HT-89 | D-Trp-Met-Asp(Ome)-Phe(3,5-DiCl)-NH2 | 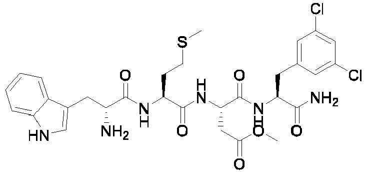 | 3.21E-06 | 3.62E-06 | 44.86 | N.A. | N.A. |
| HT-90 | D-Trp-Met-Asp(Ome)-Phe(3,5-DiF)-NH2 | 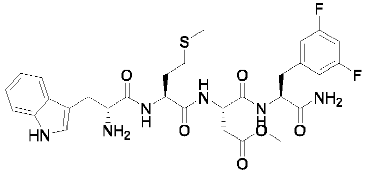 | 9.30E-06 | 5.91E-06 | 59.76 | N.A. | N.A. |
| HT-91 | D-Trp-Met-Asp(Ome)-Phe(3,4,5-TriF)-NH2 | 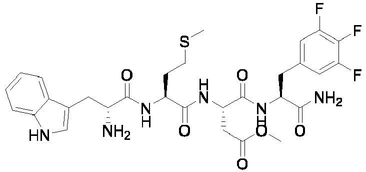 | N.A. | N.A. | 3.12 | N.A. | N.A. |
| HT-92 | D-Trp-Met-Asp(EDAN S)-Phe(2-NO2)-NH2 | 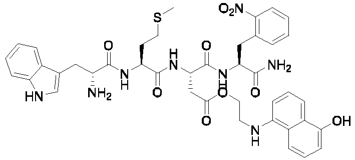 | N.A. | N.A. | -0.46 | N.A. | N.A. |
| HT-93 | D-Trp-Met-Asp(EDAN S)-Phe(2-F)-NH2 | 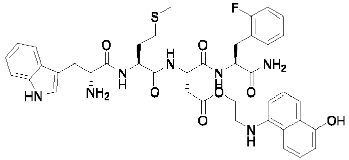 | N.A. | N.A. | -8.42 | N.A. | N.A. |
| HT-94 | D-Trp-Met-Asp(EDAN S)-Phe(3-Cl)-NH2 | 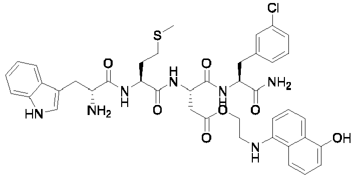 | N.A. | N.A. | 4.60 | N.A. | N.A. |
| HT-95 | D-Trp-Met-Asp(EDAN S)-Phe(3-F)-NH2 | 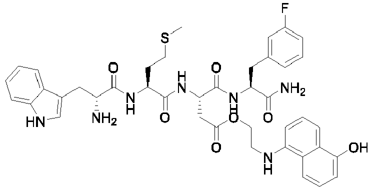 | N.A. | N.A. | 10.52 | N.A. | N.A. |
| HT-96 | D-Trp-Met-Asp(EDAN S)-Phe(3-I)-NH2 | 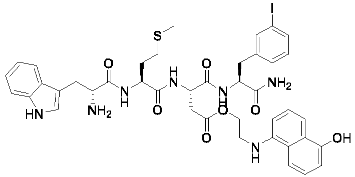 | N.A. | N.A. | 5.10 | N.A. | N.A. |
| HT-97 | D-Trp-Met-Asp(EDAN S)-Phe(3-Br)-NH2 | 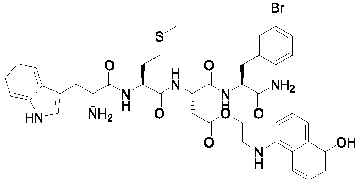 | N.A. | N.A. | 4.67 | N.A. | N.A. |
| HT-98 | D-Trp-Met-Asp(EDAN S)-Phe(4-Me)-NH2 | 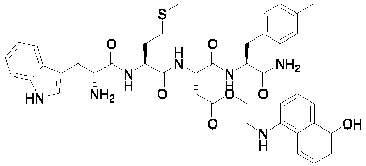 | N.A. | N.A. | 3.54 | N.A. | N.A. |
| HT-99 | D-Trp-Met-Asp(EDAN S)-Phe(4-N3)-NH2 | 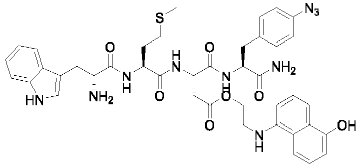 | N.A. | N.A. | 4.81 | N.A. | N.A. |
| HT-100 | wD-Trp-Met-Asp(EDAN S)-Phe(4-CN)-NH2 | 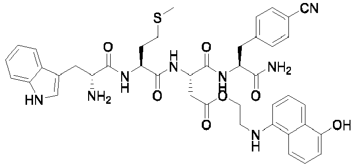 | N.A. | N.A. | 8.55 | N.A. | N.A. |
| HT-101 | D-Trp-Met-Asp(EDAN S)-Phe(4-NO2)-NH2 | 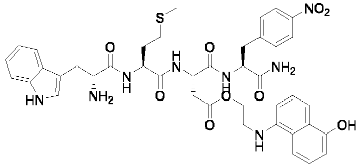 | N.A. | N.A. | -1.10 | N.A. | N.A. |
| HT-102 | D-Trp-Met-Asp(EDAN S)-Phe(4-Br)-NH2 | 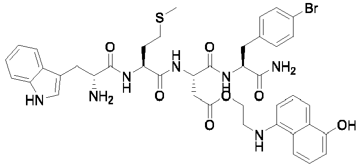 | N.A. | N.A. | -2.74 | N.A. | N.A. |
| HT-103 | D-Trp-Met-Asp(EDAN S)-Phe(4-Cl)-NH2 | 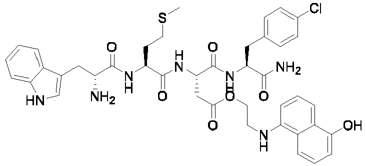 | N.A. | N.A. | 1.03 | N.A. | N.A. |
| HT-104 | D-Trp-Met-Asp(EDAN S)-Phe(4-F)-NH2 | 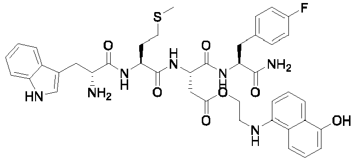 | N.A. | N.A. | -0.28 | N.A. | N.A. |
| HT-105 | D-Trp-Met-Asp(EDAN S)-Phe(4-I)-NH2 | 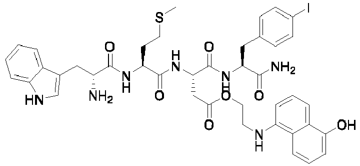 | N.A. | N.A. | 4.03 | N.A. | N.A. |
| HT-106 | D-Trp-Met-Asp(EDAN S)-Phe(3,5-DiCl)-NH2 | 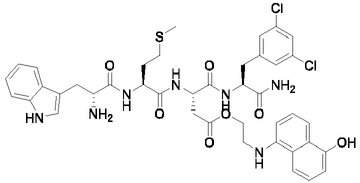 | N.A. | N.A. | 3.74 | N.A. | N.A. |
| HT-107 | D-Trp-Met-Asp(EDAN S)-Phe(3,5-DiF)-NH2 | 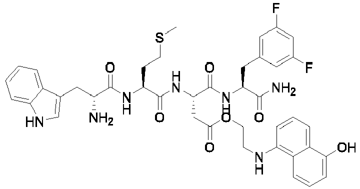 | N.A. | N.A. | 4.54 | N.A. | N.A. |
| HT-108 | D-Trp-Met-Asp(EDAN S)-Phe(3,4,5-TriF)-NH2 | 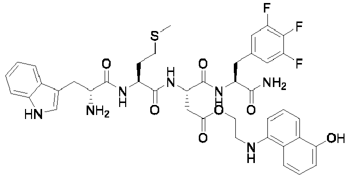 | N.A. | N.A. | 2.27 | N.A. | N.A. |
| HT-109 | D-Trp-Met(O)-Asp(Ome)-Phe(2-NO2)-NH2 | 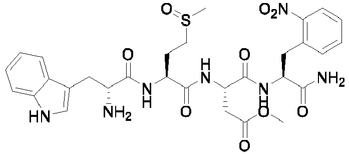 | N.A. | N.A. | 3.33 | N.A. | N.A. |
| HT-110 | D-Trp-Met(O)-Asp(Ome)-Phe(2-F)-NH2 | 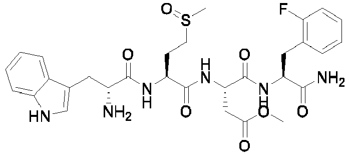 | N.A. | N.A. | 1.36 | N.A. | N.A. |
| HT-111 | D-Trp-Met(O)-Asp(Ome)-Phe(3-Cl)-NH2 | 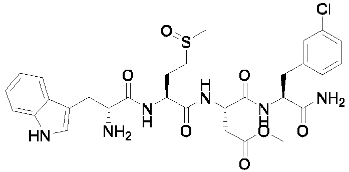 | N.A. | N.A. | -7.28 | N.A. | N.A. |
| HT-112 | D-Trp-Met(O)-Asp(Ome)-Phe(3-F)-NH2 | 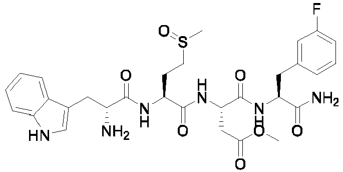 | N.A. | N.A. | 2.89 | N.A. | N.A. |
| HT-113 | D-Trp-Met(O)-Asp(Ome)-Phe(3-I)-NH2 | 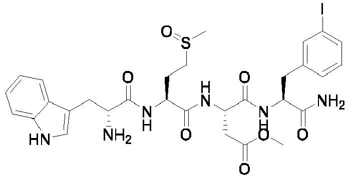 | N.A. | N.A. | 6.21 | N.A. | N.A. |
| HT-114 | D-Trp-Met(O)-Asp(Ome)-Phe(3-Br)-NH2 | 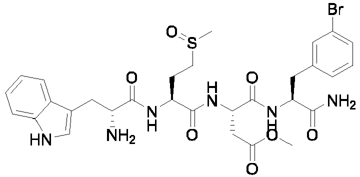 | N.A. | N.A. | -8.48 | N.A. | N.A. |
| HT-115 | D-Trp-Met(O)-Asp(Ome)-Phe(4-Me)-NH2 | 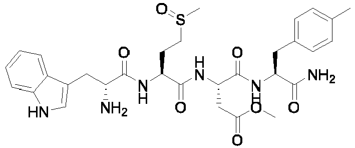 | N.A. | N.A. | 9.82 | N.A. | N.A. |
| HT-116 | D-Trp-Met(O)-Asp(Ome)-Phe(4-N3)-NH2 | 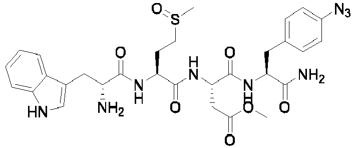 | N.A. | N.A. | 10.90 | N.A. | N.A. |
| HT-117 | D-Trp-Met(O)-Asp(Ome)-Phe(4-CN)-NH2 | 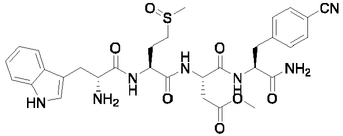 | N.A. | N.A. | 9.81 | N.A. | N.A. |
| HT-118 | D-Trp-Met(O)-Asp(Ome)-Phe(4-NO2)-NH2 | 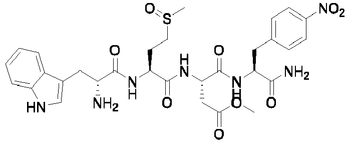 | N.A. | N.A. | 0.13 | N.A. | N.A. |
| HT-119 | D-Trp-Met(O)-Asp(Ome)-Phe(4-Br)-NH2 | 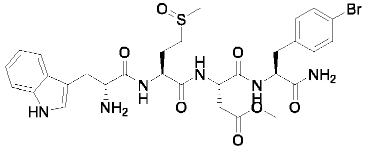 | N.A. | N.A. | 6.93 | N.A. | N.A. |
| HT-120 | D-Trp-Met(O)-Asp(Ome)-Phe(4-C1)-NH2 | 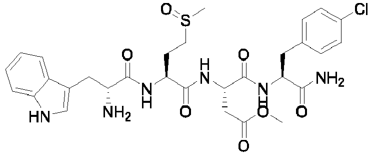 | N.A. | N.A. | -1.00 | N.A. | N.A. |
| HT-121 | D-Trp-Met(O)-Asp(Ome)-Phe(4-F)-NH2 | 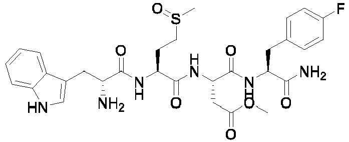 | N.A. | N.A. | 10.99 | N.A. | N.A. |
| HT-122 | D-Trp-Met(O)-Asp(Ome)-Phe(4-I)-NH2 | 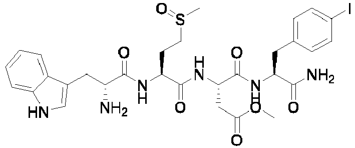 | N.A. | N.A. | 5.16 | N.A. | N.A. |
| HT-123 | D-Trp-Met(O)-Asp(Ome)-Phe(3,5-DiCl)-NH2 | 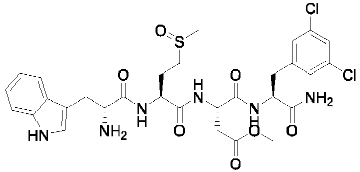 | N.A. | N.A. | 0.84 | N.A. | N.A. |
| HT-124 | D-Trp-Met(O)t-Asp(Ome)-Phe(3,5-DiF)-NH2 | 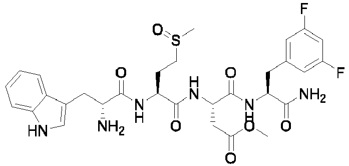 | N.A. | N.A. | 0.71 | N.A. | N.A. |
| HT-125 | D-Trp-Met(O)-Asp(Ome)-Phe(3,4,5-TriF)-NH2 | 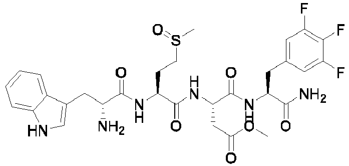 | N.A. | N.A. | -4.16 | N.A. | N.A. |
| HT-126 | D-Trp-Met(O)-Asp(Ome)-Phe(2-NO2)-NH2 | 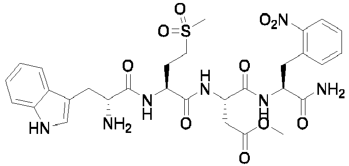 | N.A. | N.A. | 9.50 | N.A. | N.A. |
| HT-127 | D-Trp-Met(O2)-Asp(Ome)-Phe(2-F)-NH2 | 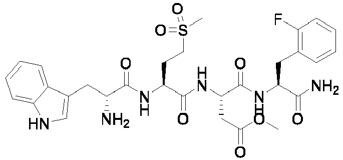 | N.A. | N.A. | 1.79 | N.A. | N.A. |
| HT-128 | D-Trp-Met(O2)-Asp(Ome)-Phe(3-Cl)-NH2 | 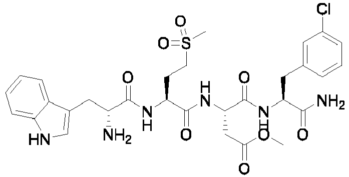 | N.A. | N.A. | 0.73 | N.A. | N.A. |
| HT-129 | D-Trp-Met(O2)-Asp(Ome)-Phe(3-F)-NH2 | 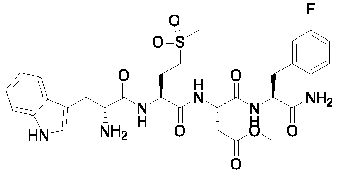 | N.A. | N.A. | -4.28 | N.A. | N.A. |
| HT-130 | D-Trp-Met(O2)-Asp(Ome)-Phe(3-I)-NH2 | 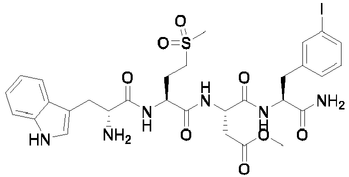 | N.A. | N.A. | 0.86 | N.A. | N.A. |
| HT-131 | D-Trp-Met(O2)-Asp(Ome)-Phe(3-Br)-NH2 | 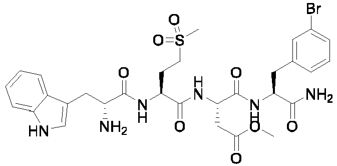 | N.A. | N.A. | 5.87 | N.A. | N.A. |
| HT-132 | D-Trp-Met(O2)-Asp(Ome)-Phe(4-Me)-NH2 | 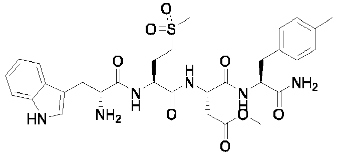 | N.A. | N.A. | -14.42 | N.A. | N.A. |
| HT-133 | D-Trp-Met(O2)-Asp(Ome)-Phe(4-N3)-NH2 | 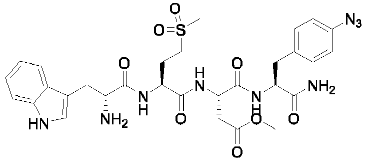 | N.A. | N.A. | 8.66 | N.A. | N.A. |
| HT-134 | D-Trp-Met(O2)-Asp(Ome)-Phe(4-CN)-NH2 | 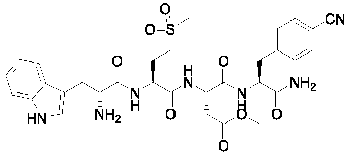 | N.A. | N.A. | 11.25 | N.A. | N.A. |
| HT-135 | D-Trp-Met(O2)-Asp(Ome)-Phe(4-NO2)-NH2 | 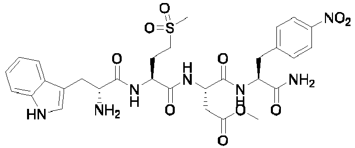 | N.A. | N.A. | 11.52 | N.A. | N.A. |
| HT-136 | D-Trp-Met(O2)-Asp(Ome)-Phe(4-Br)-NH2 | 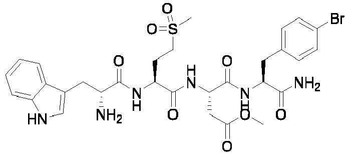 | N.A. | N.A. | 2.69 | N.A. | N.A. |
| HT-137 | D-Trp-Met(O2)-Asp(Ome)-Phe(4-C1)-NH2 | 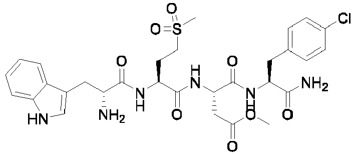 | N.A. | N.A. | 2.10 | N.A. | N.A. |
| HT-138 | D-Trp-Met(O2)-Asp(Ome)-Phe(4-F)-NH2 | 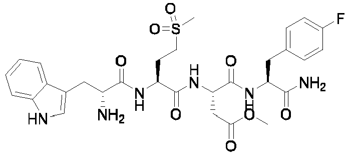 | N.A. | N.A. | -3.84 | N.A. | N.A. |
| HT-139 | D-Trp-Met(O2)-Asp(Ome)-Phe(4-I)-NH2 | 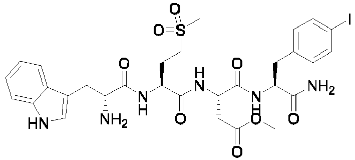 | N.A. | N.A. | 13.02 | N.A. | N.A. |
| HT-140 | D-Trp-Met(O2)-Asp(Ome)-Phe(3,5-DiCl)-NH2 | 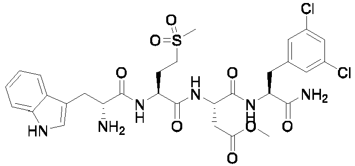 | N.A. | N.A. | 10.66 | N.A. | N.A. |
| HT-141 | D-Trp-Met(O2)-Asp(Ome)-Phe(3,5-DiF)-NH2 | 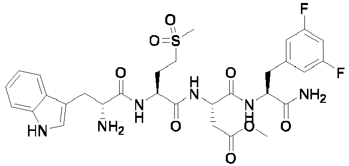 | N.A. | N.A. | 5.60 | N.A. | N.A. |
| HT-142 | D-Trp-Met(O2)-Asp(Ome)-Phe(3,4,5-TriF)-NH2 | 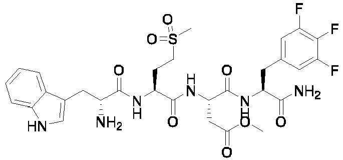 | N.A. | N.A. | 0.79 | N.A. | N.A. |
| HT-143 | D-Trp-Met(O)-Asp(EDAN S)-Phe(2-NO2)-NH2 | 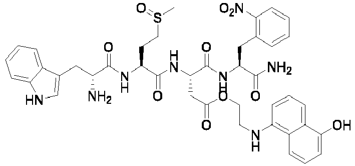 | N.A. | N.A. | 4.33 | N.A. | N.A. |
| HT-144 | D-Trp-Met(O)-Asp(EDAN S)-Phe(2-F)-NH2 | 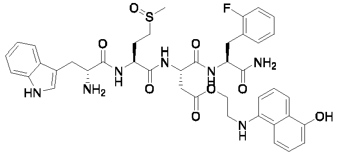 | N.A. | N.A. | 5.33 | N.A. | N.A. |
| HT-145 | D-Trp-Met(O)t-Asp(EDAN S)-Phe(3-Cl)-NH2 | 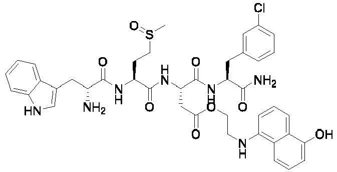 | N.A. | N.A. | -5.52 | N.A. | N.A. |
| HT-146 | D-Trp-Met(O)-Asp(EDAN S)-Phe(3-F)-NH2 | 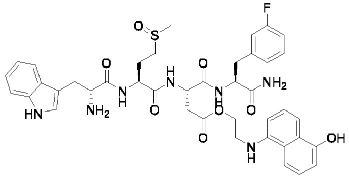 | N.A. | N.A. | 3.97 | N.A. | N.A. |
| HT-147 | D-Trp-Met(O)-Asp(EDAN S)-Phe(3-1)-NH2 | 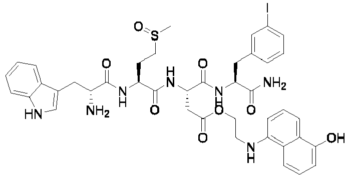 | N.A. | N.A. | 5.29 | N.A. | N.A. |
| HT-148 | D-Trp-Met(O)-Asp(EDAN S)-Phe(3-Br)-NH2 | 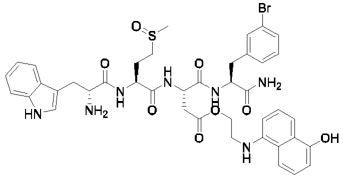 | N.A. | N.A. | 0.77 | N.A. | N.A. |
| HT-149 | D-Trp-Met(O)-Asp(EDAN S)-Phe(4-Me)-NH2 | 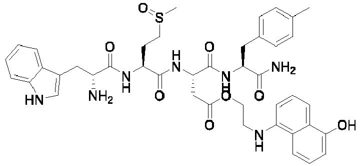 | N.A. | N.A. | 0.39 | N.A. | N.A. |
| HT-150 | D-Trp-Met(O)-Asp(EDAN S)-Phe(4-N3)-NH2 | 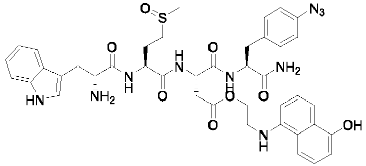 | N.A. | N.A. | 0.81 | N.A. | N.A. |
| HT-151 | D-Trp-Met(O)-Asp(EDAN S)-Phe(4-CN)-NH2 | 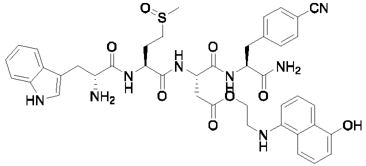 | N.A. | N.A. | 3.44 | N.A. | N.A. |
| HT-152 | D-Trp-Met(O)-Asp(EDAN S)-Phe(4-NO2)-NH2 | 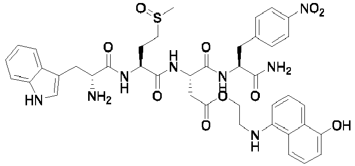 | N.A. | N.A. | 8.55 | N.A. | N.A. |
| HT-153 | D-Trp-Met(O)-Asp(EDAN S)-Phe(4-Br)-NH2 | 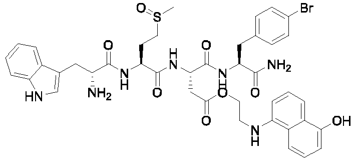 | N.A. | N.A. | -4.28 | N.A. | N.A. |
| HT-154 | D-Trp-Met(O)-Asp(EDAN S)-Phe(4-Cl)-NH2 | 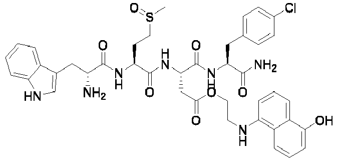 | N.A. | N.A. | -4.86 | N.A. | N.A. |
| HT-155 | D-Trp-Met(O)-Asp(EDAN S)-Phe(4-F)-NH2 | 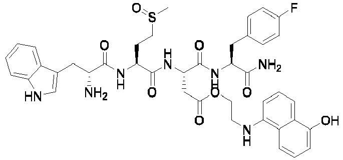 | N.A. | N.A. | 0.50 | N.A. | N.A. |
| HT-156 | D-Trp-Met(O)-Asp(EDAN S)-Phe(4-I)-NH2 | 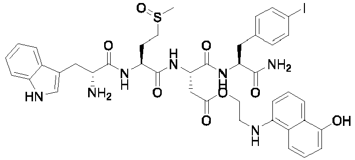 | N.A. | N.A. | -2.39 | N.A. | N.A. |
| HT-157 | D-Trp-Met(O)-Asp(EDAN S)-Phe(3,5-DiCl)-NH2 | 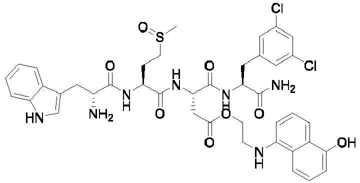 | N.A. | N.A. | 0.51 | N.A. | N.A. |
| HT-158 | D-Trp-Met(O)-Asp(EDAN S)-Phe(3,5-DiF)-NH2 | 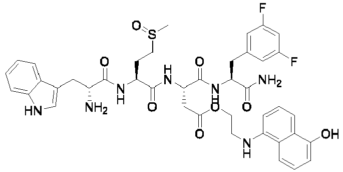 | N.A. | N.A. | -12.29 | N.A. | N.A. |
| HT-159 | D-Trp-Met(O)-Asp(EDAN S)-Phe(3,4,5-TriF)-NH2 | 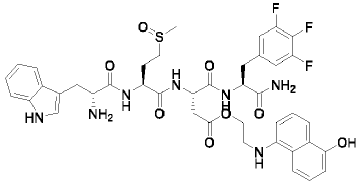 | N.A. | N.A. | 4.93 | N.A. | N.A. |
| HT-160 | D-Trp-Met(O2)-Asp(EDAN S)-Phe(2-NO2)-NH2 | 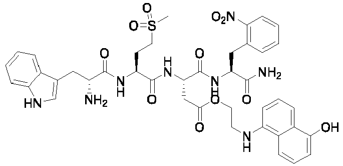 | N.A. | N.A. | 10.11 | N.A. | N.A. |
| HT-161 | D-Trp-Met(O2)-Asp(EDAN S)-Phe(2-F)-NH2 | 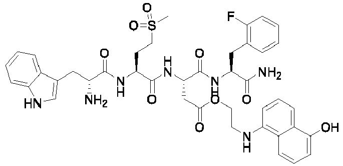 | N.A. | N.A. | 4.67 | N.A. | N.A. |
| HT-162 | D-Trp-Met(O2)-Asp(EDAN S)-Phe(3-Cl)-NH2 | 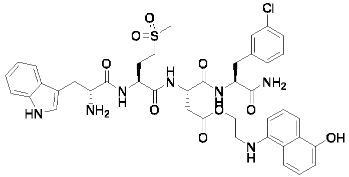 | N.A. | N.A. | -3.89 | N.A. | N.A. |
| HT-163 | D-Trp-Met(O2)-Asp(EDAN S)-Phe(3-F)-NH2 | 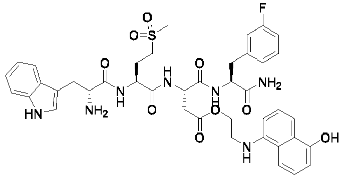 | N.A. | N.A. | 2.64 | N.A. | N.A. |
| HT-164 | D-Trp-Met(O2)-Asp(EDAN S)-Phe(3-1)-NH2 | 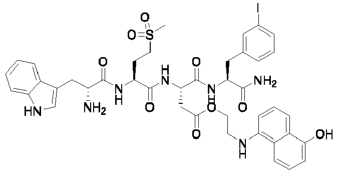 | N.A. | N.A. | 4.22 | N.A. | N.A. |
| HT-165 | D-Trp-Met(O2)-Asp(EDAN S)-Phe(3-Br)-NH2 | 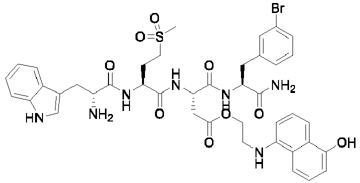 | N.A. | N.A. | 6.29 | N.A. | N.A. |
| HT-166 | D-Trp-Met(O2)-Asp(EDAN S)-Phe(4-Me)-NH2 | 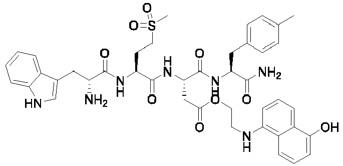 | N.A. | N.A. | 3.59 | N.A. | N.A. |
| HT-167 | D-Trp-Met(O2)-Asp(EDAN S)-Phe(4-N3)-NH2 | 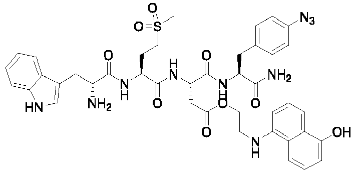 | N.A. | N.A. | 6.78 | N.A. | N.A. |
| HT-168 | D-Trp-Met(O2)-Asp(EDAN S)-Phe(4-CN)-NH2 | 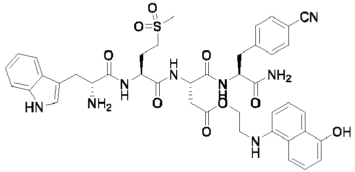 | N.A. | N.A. | 7.41 | N.A. | N.A. |
| HT-169 | D-Trp-Met(O2)-Asp(EDAN S)-Phe(4-NO2)-NH2 | 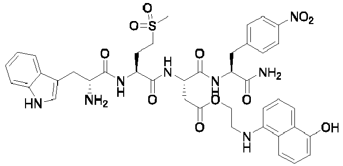 | N.A. | N.A. | 8.39 | N.A. | N.A. |
| HT-170 | D-Trp-Met(O2)-Asp(EDAN S)-Phe(4-Br)-NH2 | 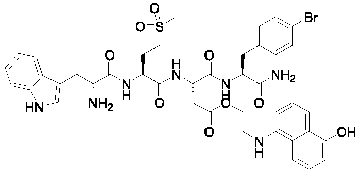 | N.A. | N.A. | 4.26 | N.A. | N.A. |
| HT-171 | D-Trp-Met(O2)-Asp(EDAN S)-Phe(4-Cl)-NH2 | 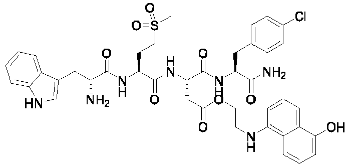 | N.A. | N.A. | -0.24 | N.A. | N.A. |
| HT-172 | D-Trp-Met(O2)-Asp(EDAN S)-Phe(4-F)-NH2 | 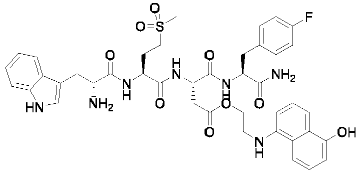 | N.A. | N.A. | -0.36 | N.A. | N.A. |
| HT-173 | D-Trp-Met(O2)-Asp(EDAN S)-Phe(4-I)-NH2 | 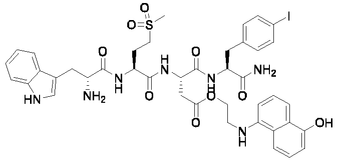 | N.A. | N.A. | 1.94 | N.A. | N.A. |
| HT-174 | D-Trp-Met(O2)-Asp(EDAN S)-Phe(3,5-DiCl)-NH2 | 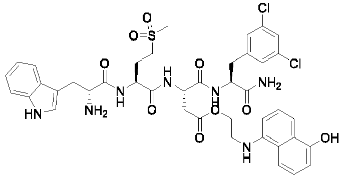 | N.A. | N.A. | -2.20 | N.A. | N.A. |
| HT-175 | D-Trp-Met(O2)-Asp(EDAN S)-Phe(3,5-DiF)-NH2 | 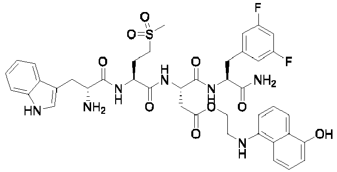 | N.A. | N.A. | -5.59 | N.A. | N.A. |
| HT-176 | D-Trp-Met(O2)-Asp(EDAN S)-Phe(3,4,5-TriF)-NH2 | 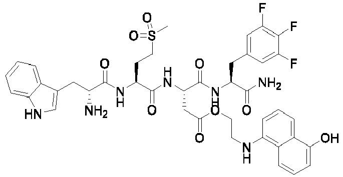 | N.A. | N.A. | -1.93 | N.A. | N.A. |
| HT-177 | Ac-Trp-Nle-Asp-Phe(3-Br)-NH2 | 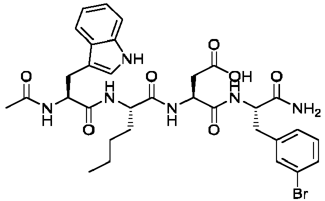 | 5.60E-11 | 2.79E-07 | N.A. | N.A. | N.A. |
| HT-178 | Ac-D-Trp-Nle-Asp-Phe(3-Br)-NH2 | 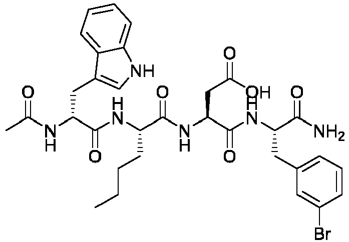 | 2.81E-09 | 5.28E-07 | N.A. | N.A. | N.A. |
| HT-179 | Ac-D-Trp-(N-Me-Nle)-Asp-Phe(3-Br)-NH2 | 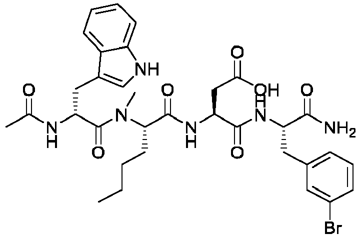 | 1.63E-06 | >10µM | N.A. | N.A. | N.A. |
| HT-180 | Ac-D-Phe-Met-Asp-Phe(3-Br)-NH2 | 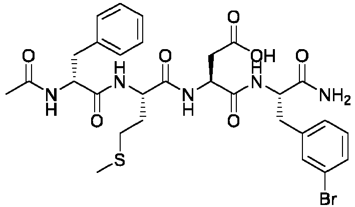 | 4.47E-07 | 3.56E-06 | N.A. | N.A. | N.A. |
| HT-181 | Ac-D-His-Met-Asp-Phe(3-Br)-NH2 | 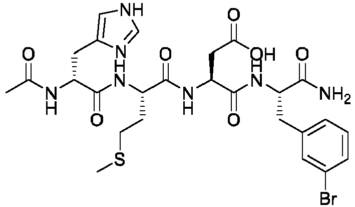 | 3.90E-06 | >10µM | N.A. | N.A. | N.A. |
| HT-182 | Ac-D-Tyr-Met-Asp-Phe(3-Br)-NH2 | 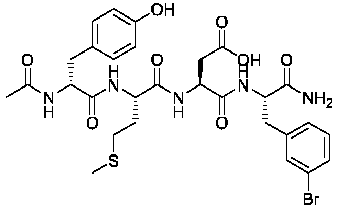 | 4.21E-07 | 3.30E-06 | N.A. | N.A. | N.A. |
| HT-183 | Ac-D-Leu-Met-Asp-Phe(3-Br)-NH2 | 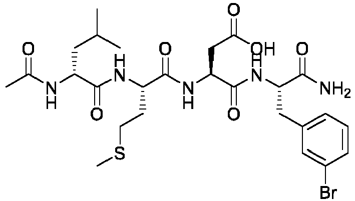 | 4.46E-06 | 9.41E-06 | N.A. | N.A. | N.A. |
| HT-184 | Ac-D-Trp-Glu-Asp-Phe(3-Br)-NH2 | 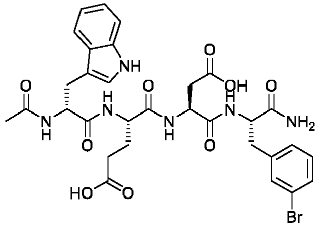 | 4.32E-06 | 4.41E-06 | N.A. | N.A. | N.A. |
| HT-185 | Ac-D-Trp-Gln-Asp-Phe(3-Br)-NH2 | 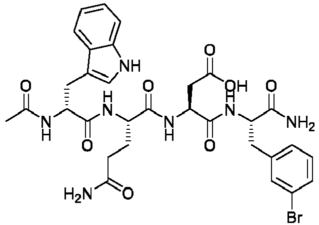 | 3.82E-06 | 1.20E-05 | N.A. | N.A. | N.A. |
| HT-186 | Ac-D-Trp-Met-Glu-Phe(3-Br)-NH2 | 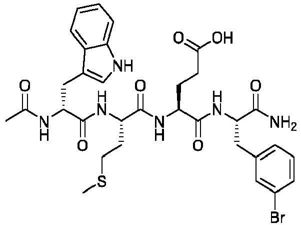 | 1.34E-06 | 7.71E-06 | N.A. | N.A. | N.A. |
| HT-187 | Ac-D-Trp-Met-Asn-Phe(3-Br)-NH2 | 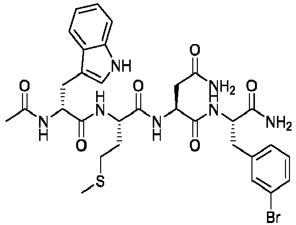 | >10µM | >10µM | N.A. | N.A. | N.A. |
| HT-188 | Ac-D-Trp-Met-Lys-Phe(3-Br)-NH2 | 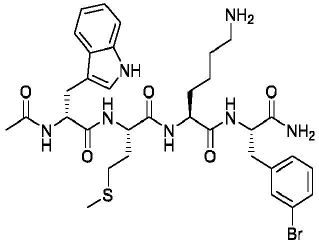 | 1.84E-06 | 7.55E-06 | N.A. | N.A. | N.A. |
| HT-189 | Ac-D-Trp-Met-His-Phe(3-Br)-NH2 | 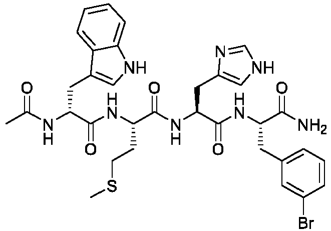 | 1.99E-06 | 5.95E-06 | N.A. | N.A. | N.A. |
| HT-190 | Ac-D-Trp-Met-Leu-Phe(3-Br)-NH2 | 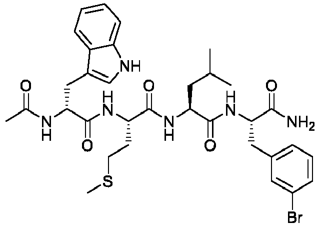 | 4.75E-06 | 6.16E-06 | N.A. | N.A. | N.A. |
| HT-191 | Ac-Trp-Met-Asp-His-NH2 | 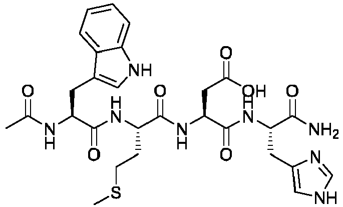 | >10µM | >10µM | N.A. | N.A. | N.A. |
| HT-192 | Ac-Trp-Met-Asp-Leu-NH2 | 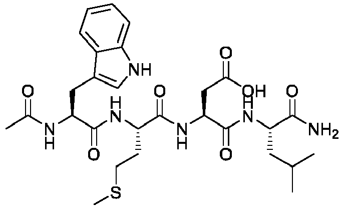 | >10µM | 8.76E-06 | N.A. | N.A. | N.A. |
| HT-193 | Ac-Trp-Met-Asp-Trp-NH2 | 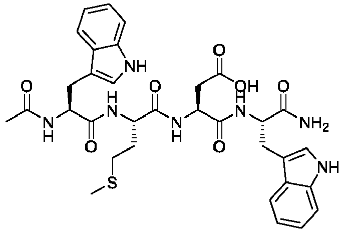 | 8.14E-10 | 5.70E-08 | N.A. | N.A. | N.A. |
| HT-194 | Ac-Trp-Met-Asp-Lys-NH2 | 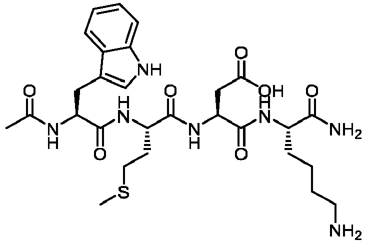 | >10µM | 2.22E-06 | N.A. | N.A. | N.A. |
| HT-195 | Ac-Trp-Met-Asp-Arg-NH2 | 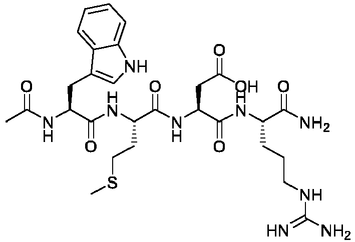 | >10µM | 2.20E-06 | N.A. | N.A. | N.A. |
| HT-196 | Ac-Trp-Met-Asp-Glu-NH2 | 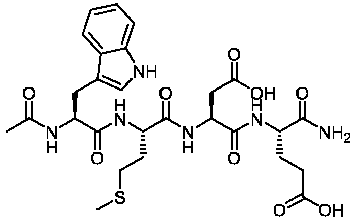 | >10µM | >10µM | N.A. | N.A. | N.A. |
| HT-197 | Ac-Trp-Met-Asp-Gln-NH2 | 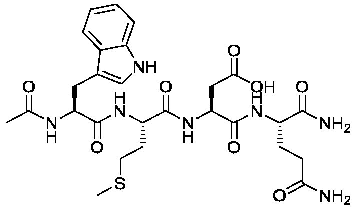 | >10µM | 1.67E-06 | N.A. | N.A. | N.A. |
| HT-198 | Fmoc-Trp-Nle-Asp-Phe(3-Br)-NH2 | 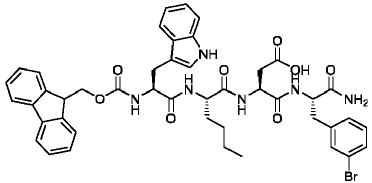 | 2.05E-07 | 9.08E-07 | N.A. | N.A. | N.A. |
| HT-199 | Fmoc-(N-Me-Trp)-Nle-Asp-Phe(3-Br)-NH2 | 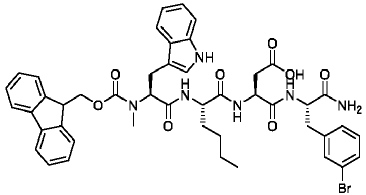 | 1.47E-09 | 6.96E-07 | N.A. | N.A. | N.A. |
| HT-200 | Fmoc-(N-Me-Trp)-(N-Me-Nle)-Asp-Phe(3-Br)-NH2 | 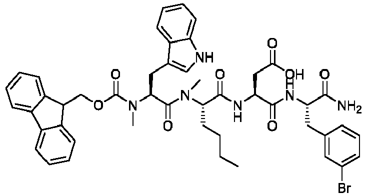 | 6.16E-07 | 1.77E-06 | N.A. | N.A. | N.A. |
| HT-201 | Ac-D-Trp-Met-Asp-Phe(3-Br)-Ala-NH2 | 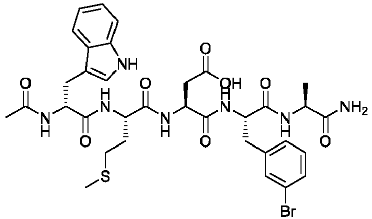 | 4.29E-06 | 3.60E-06 | N.A. | N.A. | N.A. |
| HT-202 | (Suc)-Gly-D-Trp-Nle-Asp-Phe(3-Br)-NH2 | 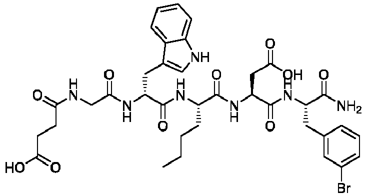 | 5.10E-09 | 1.92E-07 | N.A. | N.A. | N.A. |
| HT-203 | (Pal)-Gly-D-Trp-Nle-Asp-Phe(3-Br)-NH2 | 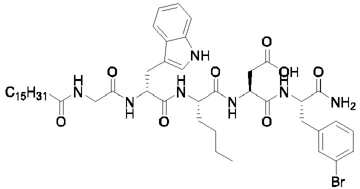 | 3.60E-08 | 1.59E-06 | N.A. | N.A. | N.A. |
| HT-204 | (Hexadecan edioic acid)-D-Trp-Nle-Asp-Phe(3-Br)-NH2 | 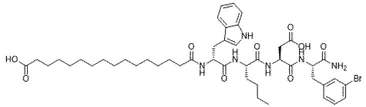 | 4.98E-08 | 2.65E-06 | N.A. | N.A. | N.A. |
| HT-205 | (Hexadecan edioic acid)-Gly-D-Trp-Nle-Asp-Phe(3-Br)-NH2 | 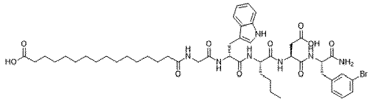 | 1.56E-07 | 5.88E-07 | N.A. | N.A. | N.A. |
| HT-206 | Ac-Asp-Tyr-Met-Gly-NH2 | 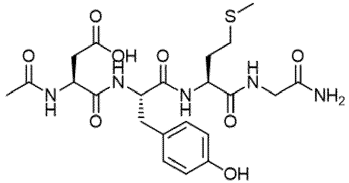 | >10µM | >10µM | N.A. | N.A. | N.A. |
| HT-207 | Ac-Tyr-Met-Gly-Trp-NH2 | 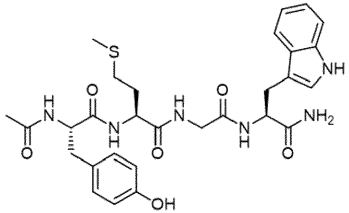 | >10µM | >10µM | N.A. | N.A. | N.A. |
| HT-208 | Ac-Asp-Tyr(SO3H)-Met-Gly-NH2 | 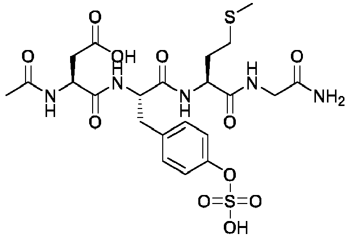 | >10µM | >10µM | N.A. | N.A. | N.A. |
| HT-209 | Ac-Tyr(SO3H)-Met-Gly-Trp-NH2 | 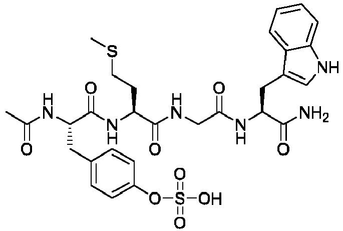 | >10µM | 2.15E-06 | N.A. | N.A. | N.A. |
| HT-210 | Ac-Met-Gly-Trp-Met-NH2 | 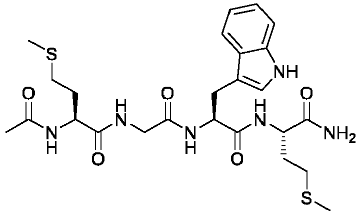 | 3.98E-07 | 6.45E-06 | N.A. | N.A. | N.A. |
| HT-211 | Ac-Gly-Trp-Met-Asp-NH2 | 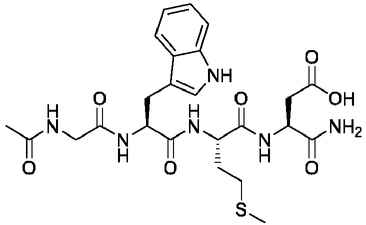 | >10µM | >10µM | N.A. | N.A. | N.A. |
| HT-212 | Ac-Nle-Gly-Trp-Met-NH2 | 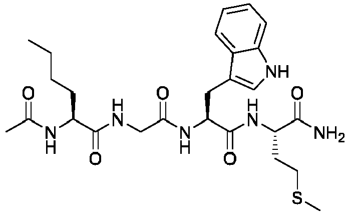 | >10µM | >10µM | N.A. | N.A. | N.A. |
| HT-213 | Ac-Nle-Gly-Trp-Nle-NH2 | 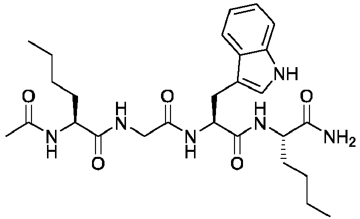 | 5.33E-07 | 4.92E-06 | N.A. | N.A. | N.A. |
| HT-214 | Ac-Gly-Trp-Nle-Asp-NH2 | 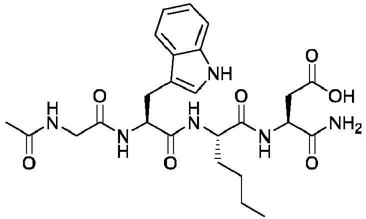 | >10µM | >10µM | N.A. | N.A. | N.A. |
| HT-215 | Ac-Gly-Trp-Nle-Asp-Phe(3-Br)-NH2 | 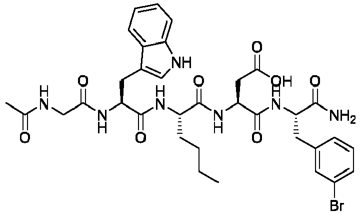 | <0.1 nm | 2.37E-07 | N.A. | N.A. | N.A. |
| HT-216 | Ac-Asp-Tyr(SO3H)-Met-Gly-Trp-NH2 | 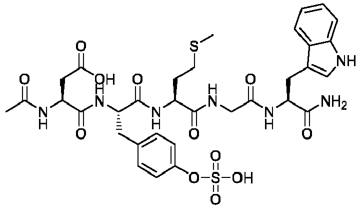 | 5.88E-07 | 1.44E-06 | N.A. | N.A. | N.A. |
| HT-217 | Ac-Leu-Ser-His-His-Glu-Arg-Leu-Lys-Cys-Asp-Glu-Trp-SerVal-Asn-Ser-Val-Gly-Lys-Ile-Glu-Cys-Val-Ser-Ala-D-Trp-Met-Asp-Phe(3-Br)-NH2 | 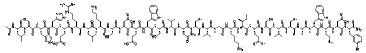 | 4.65E-07 | >10µM | N.A. | N.A. | N.A. |
| HT-218 | Ac-Tyr-Ala-Asn-Ser-Gly-His-Val-Asn-Ala-Val-Lys-Asn-Tyr-Gly-D-Trp-Met-Asp-Phe(3-Br)-NH2 | 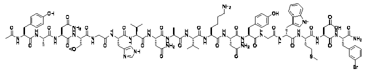 | 3.95E-07 | 6.80E-07 | N.A. | N.A. | N.A. |
| HT-219 | Ac-Glu-Ser-Glu-Leu-Thr-Asn-Glu-Ile-Pro-Ala-Glu-D-Trp-Met-Asp-Phe(3-Br)-NH2 | 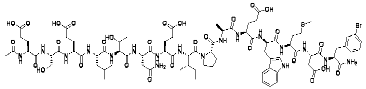 | 3.27E-07 | 2.58E-06 | N.A. | N.A. | N.A. |
| HT-220 | Ac-Thr-Lys-His-Arg-Arg-Lys-Gly-Ser-His-Gly-Leu-Glu-D-Trp-Met-Asp-Phe(3-Br)-NH2 | 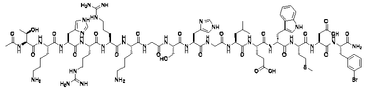 | 8.39E-06 | 2.88E-06 | N.A. | N.A. | N.A. |
| HT-221 | Biotin-D-Trp(Boc)-(N-Me-Nle)-Asp-Phe(3-Br)-NH2 | 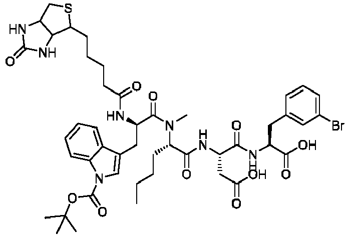 | >10µM | >10µM | N.A. | N.A. | N.A. |
| HT-222 | Biotin-D-Trp-(N-Me-Nle)-Asp-Phe(3-Br)-NH2 | 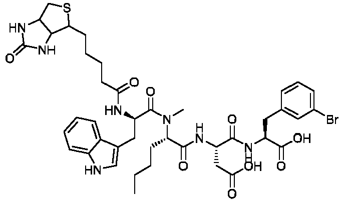 | 6.24E-08 | 1.90E-07 | N.A. | N.A. | N.A. |
| HT-223 | Ac-Phe-Met-Asp-Phe(3-Br)-N H2 | 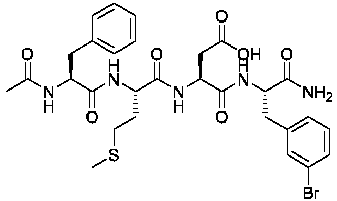 | 2.25E-07 | 1.88E-06 | N.A. | N.A. | N.A. |
| HT-224 | Ac-Phe(3-Br)-Met-Asp-Phe(3-Br)-N H2 | 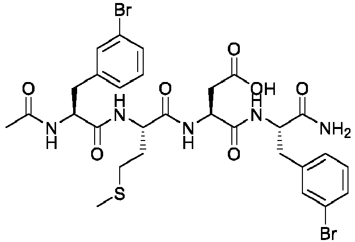 | 2.54E-08 | 8.03E-08 | N.A. | N.A. | N.A. |
| HT-225 | Ac-D-Trp-Nle-Asp-Trp-NH2 | 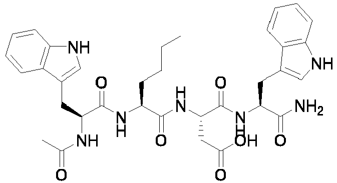 | 3.32E-08 | 4.75E-07 | N.A. | N.A. | N.A. |
| HT-226 | Ac-D-Trp-Nle-Asp-(5-HTP)-NH2 | 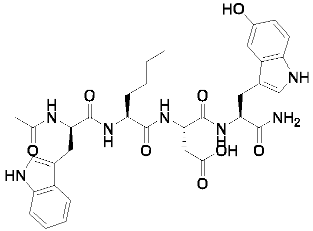 | 1.42E-05 | >10µM | N.A. | N.A. | N.A. |
| HT-227 | Ac-(5-HTP)-Nle-Asp-Phe(3-Br)-NH2 | 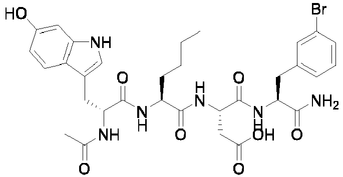 | 2.20E-08 | 3.40E-06 | N.A. | N.A. | N.A. |
| HT-228 | Ac-D-Trp-Nle-Asp-(1-Me-Trp)-NH2 | 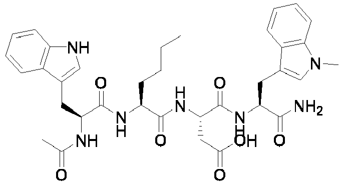 | 2.62E-07 | 3.07E-06 | N.A. | N.A. | N.A. |
| HT-229 | Ac-D-Trp-Nle-Asp-(2-Me-Trp)-NH2 | 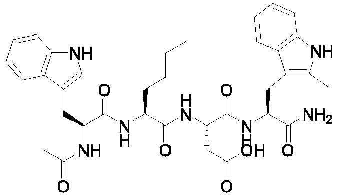 | 8.20E-06 | >10µM | N.A. | N.A. | N.A. |
| HT-230 | Ac-(1-Me-Trp)-Nle-Asp-Phe(3-Br)-NH2 1-Me-W:1-Me-Trp/Nle: norleucine | 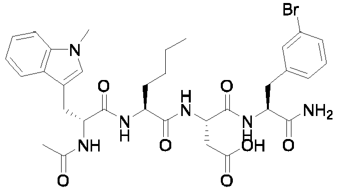 | 1.92E-09 | 5.75E-08 | N.A. | N.A. | N.A. |
| HT-231 | Ac-(2-Me-Trp)-Nle-Asp-Phe(3-Br)-NH2 | 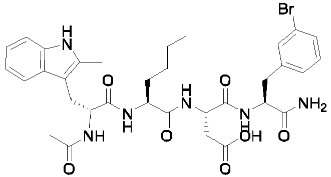 | 2.52E-07 | 1.43E-06 | N.A. | N.A. | N.A. |
| HT-232 | Ac- (1-Me-Trp) -Nle-Asp-(1-Me-Trp)-NH2 | 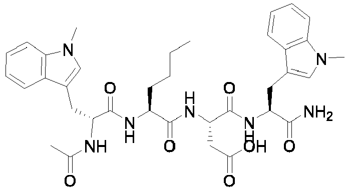 | 3.76E-07 | 3.28E-06 | N.A. | N.A. | N.A. |
| HT-233 | Ac- (1-Me-Trp) -Nle-Asp-(2-Me-Trp)-NH2 | 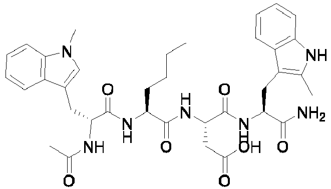 | >10µM | 3.65E-06 | N.A. | N.A. | N.A. |
| HT-234 | Ac-(2-Me-Trp)-Nle-Asp-(1-Me-Trp)-NH2 | 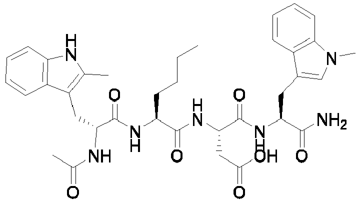 | 5.46E-06 | 4.00E-06 | N.A. | N.A. | N.A. |
| HT-235 | Ac-(2-Me-Trp)-Nle-Asp-(2-Me-Trp)-NH2 | 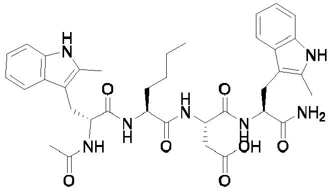 | >10µM | >10µM | N.A. | N.A. | N.A. |
| HT-236 | Ac-Phe(3-Br)-Nle-Asp-Phe(3-Br)-NH2 | 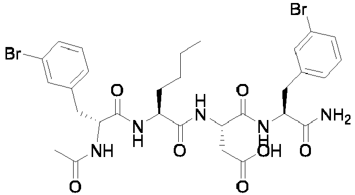 | 9.77E-08 | 3.57E-07 | N.A. | N.A. | N.A. |
| HT-237 | Ac-Phe(3-Br)-Nle-Glu-Phe(3-Br)-NH2 | 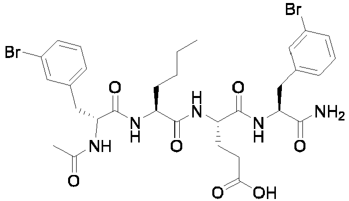 | >10µM | >10µM | N.A. | N.A. | N.A. |
| HT-238 | Ac-D-Trp-Nle-Glu-Phe(3-Br)-NH2 | 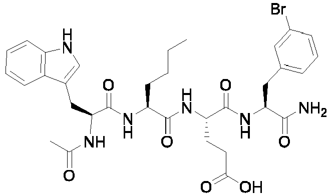 | 3.44E-08 | 2.82E-05 | N.A. | N.A. | N.A. |
| HT-239 | Ac-D-Trp-Nle-Glu-(5-HTP)-NH2 | 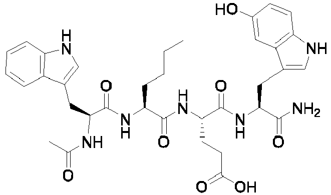 | >10µM | >10µM | N.A. | N.A. | N.A. |
| HT-240 | Ac-(5-HTP)-Nle-Glu-Phe(3-Br)-NH2 | 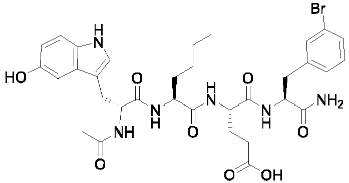 | 5.27E-07 | >10µM | N.A. | N.A. | N.A. |
| HT-241 | Ac-D-Trp-Nle-Glu-(1-Me-Trp)-NH2 | 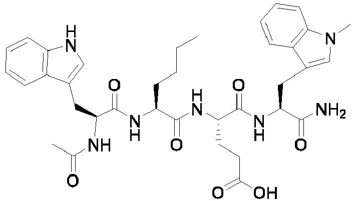 | 3.55E-06 | >10µM | N.A. | N.A. | N.A. |
| HT-242 | Ac-D-Trp-Nle-Glu-(2-Me-Trp)-NH2 | 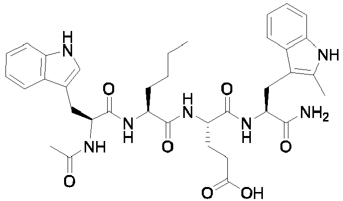 | >10µM | 1.31E-06 | N.A. | N.A. | N.A. |
| HT-243 | Ac-(1-Me-Trp)-Nle-Glu-Phe(3-Br)-NH2 | 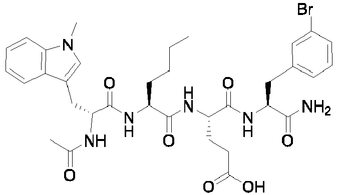 | 4.40E-07 | 1.41E-06 | N.A. | N.A. | N.A. |
| HT-244 | Ac-(2-Me-Trp)-Nle-Glu-Phe(3-Br)-NH2 | 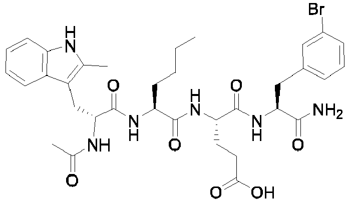 | 7.32E-06 | 1.36E-06 | N.A. | N.A. | N.A. |
| HT-245 | Ac- (1-Me-Trp) -Nle-Glu- (1-Me-Trp)-NH2 | 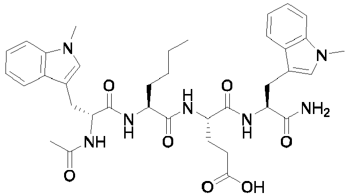 | >10µM | 1.76E-06 | N.A. | N.A. | N.A. |
| HT-246 | Ac- (1-Me-Trp) -Nle-Glu- (2-Me-Trp)-NH2 | 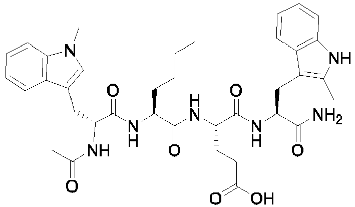 | >10µM | 2.13E-06 | N.A. | N.A. | N.A. |
| HT-247 | Ac-(2-Me-Trp)-Nle-Glu- (1-Me-Trp)-NH2 | 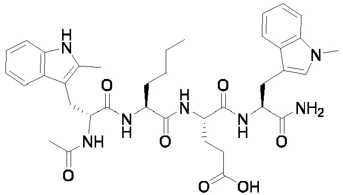 | 1.90E-06 | 6.20E-07 | N.A. | N.A. | N.A. |
| HT-248 | Ac-(2-Me-Trp)-Nle-Glu- (2-Me-Trp)-NH2 | 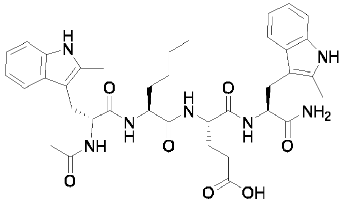 | 3.31E-08 | 4.70E-07 | N.A. | N.A. | N.A. |
| HT-249 | Fmoc-(N-Me-D-Trp)-Nle-Asp-Phe(3-Br)-NH2 | 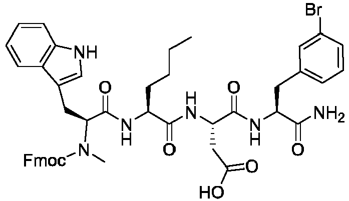 | 2.263E-09 | 4.55E-06 | N.A. | N.A. | N.A. |
| HT-250 | Fmoc-Gly-(N-Me-D-Trp)-Nle-Asp-Phe(3-Br)-NH2 | 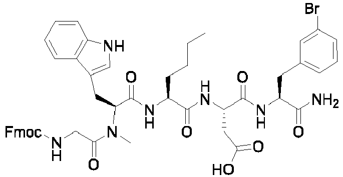 | 3.86E-07 | 2.85E-06 | N.A. | N.A. | N.A. |
| HT-251 | Fmoc-Gly-(N-Me-Trp)-Nle-Asp-Phe(3-Br)-NH2 | 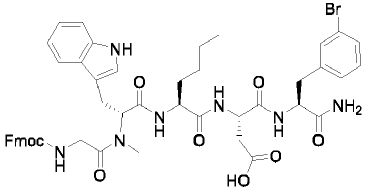 | 5.924E-09 | 1.61E-06 | N.A. | N.A. | N.A. |
| HT-252 | Fmoc-(N-Me-Gly)-(N-Me-Trp)-Nle-Asp-Phe(3-Br)-NH2 | 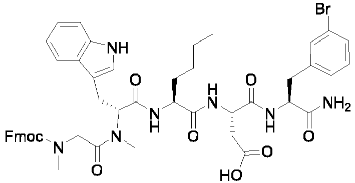 | 2.55E-07 | 1.03E-06 | N.A. | N.A. | N.A. |
| HT-253 | Fmoc-(N-Me-Trp)-Nle-Asp(O-Me)-Phe(3-Br)-NH2 | 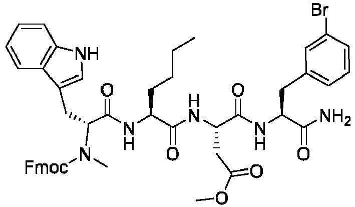 | 7.44E-07 | 1.92E-06 | N.A. | N.A. | N.A. |
| HT-254 | Fmoc-(N-Me-Trp)-Pa-Asp(O-Me)-Phe(3-Br)-NH2 | 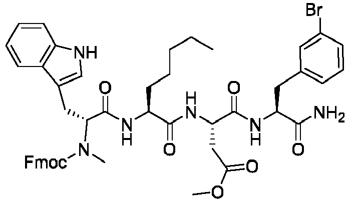 | 2.255E-08 | 1.04E-06 | N.A. | N.A. | N.A. |
| HT-255 | Glu-D-Trp-Met-Asp-Phe(3-Br)-NH2 | 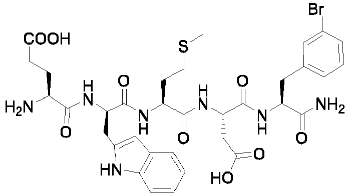 | 5.69E-10 | 4.03E-08 | N.A. | N.A. | N.A. |
| HT-256 | Sia-D-Trp-Met-Asp-Phe(3-Br)-NH2 | 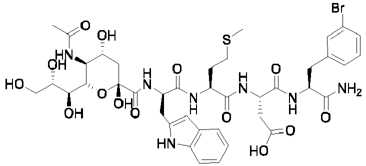 | 6.72E-10 | 4.19E-08 | N.A. | N.A. | N.A. |
| HT-257 | Ac-D-Trp-Pa-Asp-Phe(3-Br)-NH2 | 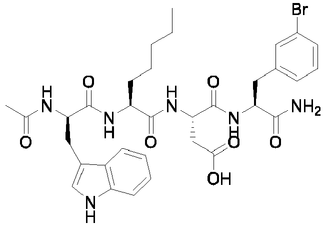 | 3.34E-09 | 1.32E-07 | N.A. | N.A. | N.A. |
| HT-258 | Fmoc-(N-Me-Trp)-Pa-Asp-Phe(3-Br)-NH2 | 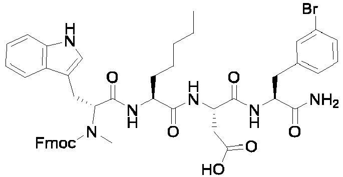 | 2.255E-08 | 1.04E-06 | N.A. | N.A. | N.A. |
| HT-259 | Ac-D-Trp-Oa-Asp-Phe(3-Br)-NH2 Oa: 2-aminooctan oic acid | 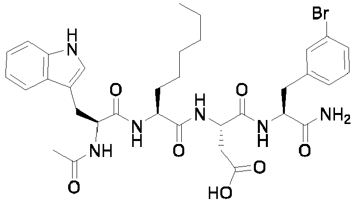 | 2.29E-09 | 5.92E-08 | N.A. | N.A. | N.A. |
| HT-260 | Ac-D-Trp-Na-Asp-Phe(3-Br)-NH2 | 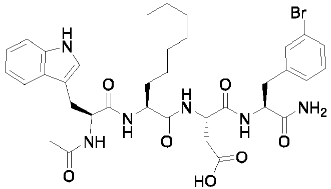 | 1.60E-07 | 2.74E-06 | N.A. | N.A. | N.A. |
| HT-261 | Ac-D-Trp-Da-Asp-Phe(3-Br)-NH2 | 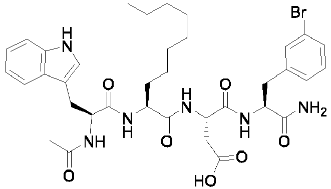 | 3.62E-07 | 3.82E-06 | N.A. | N.A. | N.A. |
| HT-262 | Ac-(2-Me-Trp)-Nle-Aa-Phe(3-Br)-NH2 | 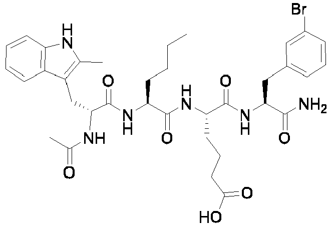 | 1.18E-06 | >10µM | N.A. | N.A. | N.A. |
| HT-263 | Ac-Oa-Nle-Glu-Phe(3-Br)-NH2 | 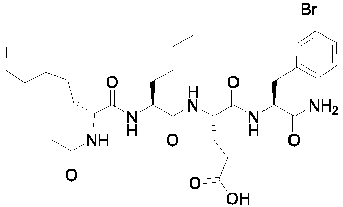 | 6.45E-05 | >10µM | N.A. | N.A. | N.A. |
| HT-264 | Ac-Na-Nle-Glu-Phe(3-Br)-NH2 | 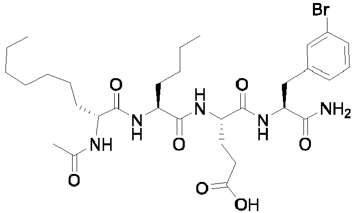 | 1.10E-05 | >10µM | N.A. | N.A. | N.A. |
| HT-265 | Ac-Da-Nle-Glu-Phe(3-Br)-NH2 Da: 2-aminodecan oic acid | 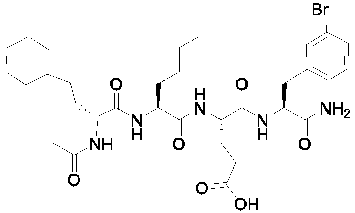 | 1.30E-05 | >10µM | N.A. | N.A. | N.A. |
| HT-266 | Ac-Ha-Nle-Oa-Phe(3-Br)-NH2 | 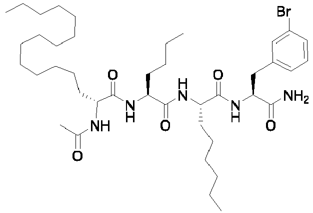 | 4.02E-08 | 1.29E-06 | N.A. | N.A. | N.A. |
| HT-267 | Ac-D-Trp-Met-Asp-Phe(3-Br)-NH2 | 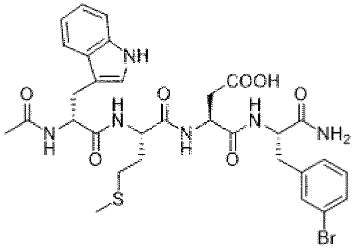 | 1.13E-10 | 2.38E-08 | N.A. | N.A. | N.A. |
| HT-268 | Fmoc-(N-Me-Trp)-Nle-Asp-Phe(3-Br)-NH2 | 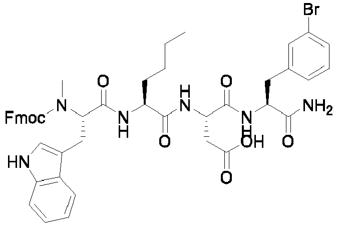 | 1.30E-09 | 4.97E-08 | N.A. | N.A. | N.A. |
| HT-269 | Ac-(N-Me-Trp)-Nle-Asp-Phe(3-Br)-NH2 | 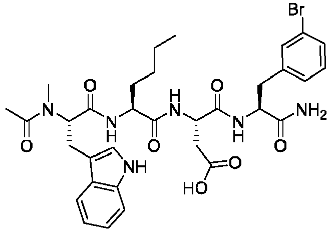 | N.A. | N.A. | N.A. | 33.52 | 0.05 |
| HT-270 | Ac-D-Trp-Nle-Aa-Phe(3-Br)-NH2 | 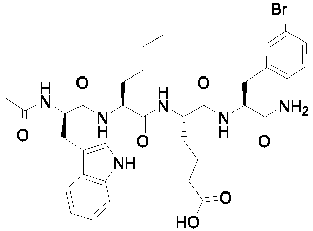 | N.A. | N.A. | N.A. | 80.85 | -0.03 |
| HT-271 | Ac-Trp-Nle-Aa-Phe(3-Br)-NH2 | 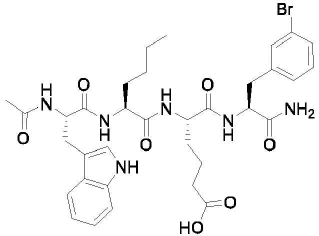 | N.A. | N.A. | N.A. | 104.56 | 40.66 |
| HT-272 | Ac-(N-Me-Trp)-Nle-Aa-Phe(3-Br)-NH2 | 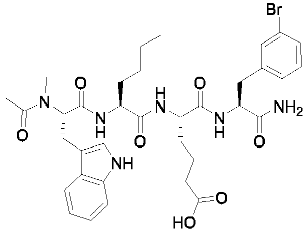 | N.A. | N.A. | N.A. | 86.09 | -0.33 |
| HT-273 | Ac-(N-Me-Trp)-Nle-Asp-Phe(3-Br)-NH2 | 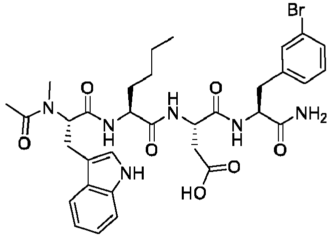 | N.A. | N.A. | N.A. | 96.54 | 52.14 |
| HT-274 | Sia-(N-Me-Trp)-Nle-Asp-Phe(3-Br)-NH2 | 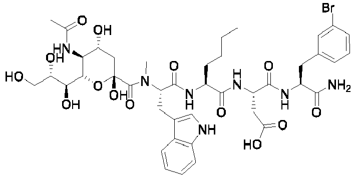 | N.A. | N.A. | N.A. | 72.88 | 0.10 |
| HT-275 | Sia-(N-Me-Trp)-Nle-Aa-Phe(3-Br)-NH2 | 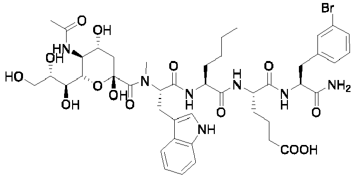 | N.A. | N.A. | N.A. | 100.24 | 62.28 |
| HT-276 | Glu-(N-Me-Trp)-Nle-Asp-Phe(3-Br)-NH2 | 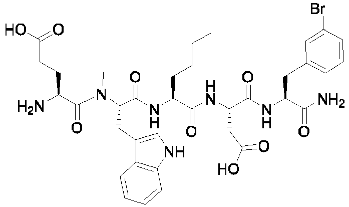 | N.A. | N.A. | N.A. | 101.04 | 4.91 |
| HT-277 | Glu-(N-Me-Trp)-Nle-Aa-Phe(3-Br)-NH2 | 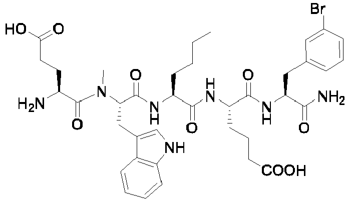 | N.A. | N.A. | N.A. | 13.46 | -0.13 |
| HT-278 | Sia-(N-Me-Trp)-Nle-Asp-Phe(3-Br)-NH2 | 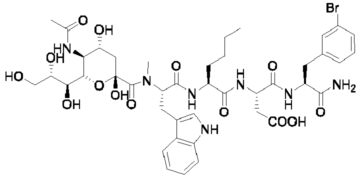 | N.A. | N.A. | N.A. | 93.12 | 40.85 |
| HT-279 | Cbz-(1-Me-Trp) -Oa-Asp-Phe(3-Br)-NH2 | 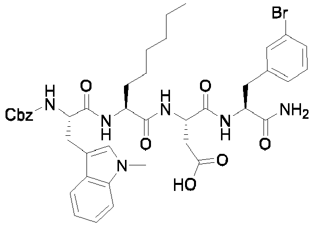 | N.A. | N.A. | N.A. | 90.99 | -0.19 |
| HT-280 | Ac-Met-Asp-Phe-NH2 | 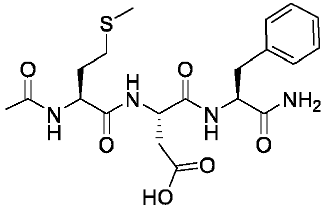 | N.A. | N.A. | N.A. | -0.16 | -0.33 |
| HT-281 | Ac-Oa-Asp-Phe(3-Br)-NH2 | 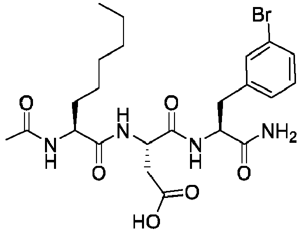 | N.A. | N.A. | N.A. | 0.03 | -0.09 |
| HT-282 | Ac-Met-Asp-Phe(3-Br)-NH2 | 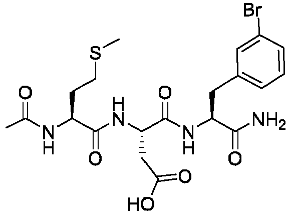 | N.A. | N.A. | N.A. | 0.45 | 0.13 |
| HT-283 | Ac-Nle-Asp-Phe(3-Br)-NH2 | 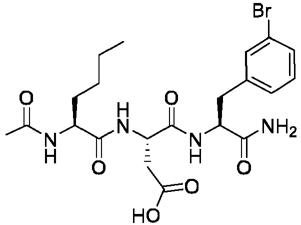 | N.A. | N.A. | N.A. | -0.03 | -0.04 |
| HT-284 | Ac-Asp-Phe-NH2 | 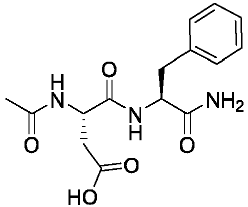 | N.A. | N.A. | N.A. | 0.09 | 0.01 |
| HT-285 | Asp-Tyr-Met-Gly-Trp-Met-Asp-Phe-NH2 | 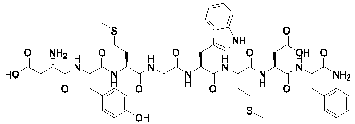 | 1.38E-09 | N.A. | N.A. | 92.06 | N.A. |
| HT-286 | His-His-His-His-His-His-Ser-Ser-Gly-Gly-Trp-Lys-Asp-Phe-NH2 | 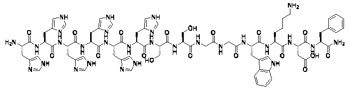 | N.A. | N.A. | N.A. | -0.52 | -0.40 |
| HT-287 | His-His-His-His-His-His-Ser-Ser-Gly-Gly-Asp-Tyr-Met-Gly-Trp-Lys-Asp-Phe-NH2 | 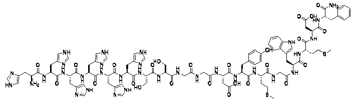 | N.A. | N.A. | N.A. | 99.10 | 77.83 |
| HT-288 | D-Trp-Lys-Asp-Phe(3-Br)-D-Ala-NH2 | 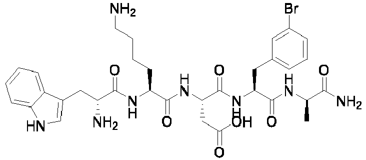 | >10 µM | N.A. | N.A. | N.A. | N.A. |
| HT-289 | D-Trp-Val-Asp-Phe(3-Br)-D-Ala-NH2 | 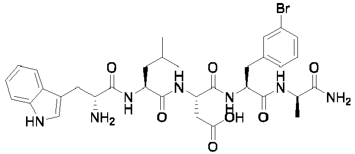 | 3.19E-06 | N.A. | N.A. | N.A. | N.A. |
| HT-290 | D-Trp-Met-Asp-Phe(3-Br)-D-Ala-NH2 | 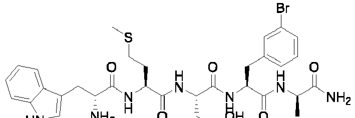 | 6.68E-06 | N.A. | N.A. | N.A. | N.A. |
| HT-291 | cyclo(Trp-Met-Asp-Phe(3-Br)-Gly-Gly-Gly-Gly) | 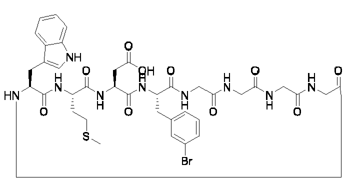 | 1.06E-05 | N.A. | N.A. | N.A. | N.A. |
| HT-292 | D-Ala-Trp-Met-Asp-Phe(3-Br)-D-Ala-NH2 | 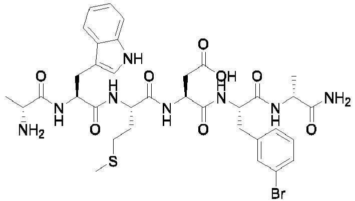 | 1.45E-06 | N.A. | N.A. | N.A. | N.A. |

| | | | | | | | |
|---|---|---|---|---|---|---|---|
| Note: NA indicates "not detected". | | | | | | | |

It can be seen from the activity data in Table 1 that the first amino acid tryptophan may be of D-type or L-type, but the activity of L-type is stronger, and the amino acids at positions 2-4 are methionine/n-leucine, aspartic acid and phenylalanine, respectively. The amino acids at positions 2-4 are L-type amino acids, which is necessary for the activity of polypeptide analogues. The activity of compound is better when R₂ is a side chain of n-leucine, and R₄ is a side chain of 3-bromophenylalanine. When R₇ is an acetyl group, the compounds such as HT-177 and HT-267 are more active.

### Example 2

In the Example, the pharmacokinetic properties of HT-267, HT-177 and CCK4 (HT-9) in KM mice (male) were determined after intravenous administration (n = 4). Specifically, the above three polypeptides were dissolved in a solution of 5% DMSO and 95% secondary deionized water, respectively, and injected into the tail vein of KM mice at a dose of 1 mg/kg, respectively. After a single dose, whole blood (100 µL) was taken from the orbital vein at different time points and the whole blood sample was collected in a heparinized tube. The plasma component was immediately separated by centrifugation and the corresponding concentrations were analyzed by LC/MS/MS.

**Table 2**

| | **HT-267 I.V.** | **HT-177 I.V.** | **CCK4 (HT-9) I.V.** |
|---|---|---|---|
| Animal Number | ♂4 | ♂4 | ♂4 |
| Dose level (mg/kg) | 1 | 1 | 1 |
| AUC(0-∞) g/L*h | 302.083 | 640.851 | ~ |
| T_{1/2} (h) | 1.601 | 0.721 | ~ |
| Tₘₐₓ (h) | 0.033 | 0.25 | ~ |
| Cₘₐₓ (µg/L) | 1929.095 | 403.377 | ~ |

| | | | |
|---|---|---|---|
| Note: ~ indicates "not detected". | | | |

The results showed that the presence of CCK4 could not be detected in blood samples after tail vein injection, indicating that its concentration was lower than the detection limitation (50 ng/ml), possibly due to the rapid decomposition of CCK4 after intravenous injection. Both compounds HT-267 and HT-177 showed a good half-life, 1.601 and 0.721 hours, respectively. At the same time, the time of Tmax was 0.033 and 0.25 hours, respectively. In addition, a good result was also shown for the maximum concentration (Cmax), especially where HT-267 reached 1929.095 µg/L.

### Example 3

After anesthetized with 100 mg/kg dose of pentobarbital, adult (eight-week) wild-type mice (C57BL/6) were gently immobilized on a digital brain stereotaxic device (RWD Life Science, China) via the mouse head and injected with AAV9-hsyn-cck-2.0-GFP (3.2×10¹² vg/ml) virus at multiple sites (250 nl/site) in the cortical surface region (DV=300 µm). For cortical imaging, a 3mm × 3mm rectangular craniotomy was performed and a coverslip was implanted as a viewing window, and the dura mater was not removed. Two weeks after viral injection, the CCK-B receptor carrying green fluorescence was expressed. When the ligand was bound to this artificial receptor, a green fluorescence signal was observed, which was monitored by a widefield fluorescence microscope, as shown in Figure 2.

The results showed that after intraperitoneal injection of compound HT-177 solution in mice that had been injected with the above virus, a green fluorescence signal was observed (Figure 2B), indicating that HT-177 drug crossed the blood-brain barrier and was bound to the CCK-B receptor expressed by the virus.

### Example 4

Adult wild-type mice (C57BL/6) were housed under standard conditions with free access to food and water. Morris Water Maze is an experimental method designed by British psychologist Morris in 1981 and applied to the study of brain learning and memory mechanism, which is widely used in scientific research in learning and memory, senile dementia, intelligence and aging, new drug development/screening/evaluation and other disciplines. Our Morris water maze experiment for testing the spatial memory ability of mice mainly included two parts: positioning navigation experiment and space exploration experiment. The positioning navigation experiment lasted for 10 days, and the mice were put into the water four times a day from four entry points facing the pool wall, and the time they spent in finding the hidden platform under the water surface (evasion latency) was recorded. The space exploration experiment was to remove the platform at 24 hours after the positioning navigation experiment, and then put the mice into the pool, record their swimming tracks within one minute, investigate the memory of the mice on the original platform, and calculate the proportion of exploration duration of the mice in the quadrant where the original platform was located (target quadrant).

Senile wild-type mice were tested for their spatial memory ability using the Morris water maze experiment, and given the CCK4 (HT-9) treatment. We randomized senile mice into two groups, one given intraperitoneal injection of the CCK4 solution and the other given intraperitoneal injection of the solvent of the drug as a control. As shown in Figure 3A, in the positioning navigation experiment, after several days of training, the evasion latency of the administration group was shorter than that of the control group, indicating that the administration group showed better learning ability than the control group. Figure 3B was track diagrams of the two groups of mice in the space exploration experiment, and the administration group showed more spatial exploration tracks at the original position of the platform, indicating that the mice had better memory backtracking ability to the platform position. This experiment showed that CCK4 produced an improvement on spatial memory deficiency in senile mice.

### Example 5

Adult male tri-transgenic Alzheimer's mice (3xTg-AD) were housed under standard conditions with free access to food and water. Our Morris water maze experiment for testing Alzheimer's mice mainly included two parts: positioning navigation experiment and space exploration experiment. The positioning navigation experiment lasted for 8-10 days, and the mice were put into the water four times a day from four entry points facing the pool wall, and the time they spent in finding the hidden platform under the water surface (evasion latency) was recorded. The space exploration experiment was to remove the platform after the positioning navigation experiment, and then put the mice into the pool, record their swimming tracks within one minute, investigate the memory of the mice on the original platform, and calculate the proportion of exploration duration of the mice in the quadrant where the original platform was located (target quadrant). Alzheimer's mice were confirmed to have memory deficiency in the Morris water maze experiment, showing not only a longer evasion latency for positioning the hidden platform in the positioning navigation experiment, but also a worse memory backtracking ability in the space exploration experiment.

We randomized Alzheimer's mice into four groups, with the first group given intraperitoneal injection of the CCK4 solution, the second group given intraperitoneal injection of the solvent of CCK4 solution as a control, the third group given intraperitoneal injection of the compound HT-267 solution, and the fourth group given intraperitoneal injection of the solvent of HT-267 solution as a control. In the positioning navigation training of the CCK4 treatment experiment, because the half-life of CCK4 was too short (less than 5 minutes), intraperitoneal injection of the drug was given before each training, and the treatment was given four times a day, and the training was carried out four times a day, for a total of 10 days. As shown in Figure 4A, after 10 days of training, the evasion latency of the CCK4 administration group was shorter than that of the control group under the same operation, indicating that the administration group showed better learning ability than the control group. Figure 4B was track heat maps of the two groups of mice in the space exploration experiment, and in the lower map the CCK4 administration group showed more spatial exploration tracks at the original position of the platform, indicating that the mice had a better memory backtracking ability to the platform position. Figure 4C compared the proportion of exploration duration in the target quadrant between the two groups of mice, and the CCK4 administration group was significantly higher than the control group.

In the positioning navigation training of the compound HT-267 treatment experiment, because HT-267 had a half-life of up to 1.601 hours, only one intraperitoneal injection was required per day, which can act on four trainings, so the treatment was given once a day, and the training was carried out four times a day, for a total of 8 days. As shown in Figure 4D, after 8 days of training, the evasion latency of the HT-267 administration group was shorter than that of the control group under the same operation, indicating that the administration group showed a better learning ability than the control group. Figure 4E was track heat maps of the two groups of mice in the space exploration experiment, and in the lower map the HT-267 administration group showed more spatial exploration tracks at the original position of the platform, indicating that the mice had a better memory backtracking ability to the platform position. Figure 4F compared the proportion of exploration duration in the target quadrant between the two groups of mice, and the HT-267 administration group was significantly higher than the control group. Comparing Figure 4F with Figure 4C, it can be seen that the HT-267 administration group performed slightly better than the CCK4 administration group, and considering that HT-267 had fewer treatment times and fewer treatment days than CCK4, we believed that HT-267 had a better efficacy than CCK4.

This experiment showed that both CCK4 and compound HT-267 produced an improvement on spatial memory deficiency in Alzheimer's mice, and HT-267 had a better efficacy.

### Example 6

Adult memory-deficient mice were housed under standard conditions with free access to food and water. Our Morris water maze experiment for testing memory-deficient mice mainly included two parts: positioning navigation experiment and space exploration experiment. The experimental methods were the same as Example 5.

We randomized memory-deficient mice into six groups, with the first group given intraperitoneal injection of the CCK4 solution, the second group given intraperitoneal injection of the solvent of CCK4 solution as a control, the third group given intraperitoneal injection of the compound HT-267 solution, the fourth group given intraperitoneal injection of the compound HT-177 solution, the fifth group given intraperitoneal injection of the compound HT-178 solution, and the sixth group given intraperitoneal injection of the solvent of compound solution as a control. In the positioning navigation experiment, the treatment and training methods of the first and the second groups were the same as those of the first and second groups in Example 5, that is, four times of treatment a day, four times of training a day, for a total of 10 days of training. The treatment and training methods of the third to sixth groups were the same as the third and fourth groups in Example 5, that is, one treatment a day, four times of training a day, for a total of 8 days of training. In the space exploration experiment, the spatial memory ability of mice was tested in the same way for each group.

Figure 5A compared the proportion of exploration duration of each group of mice in the target quadrant in the space exploration experiment, and it can be seen from Figure 5A that the proportion of exploration duration in the CCK4 treatment group was significantly higher than that in the control group, indicating that CCK4 produced an improvement on the spatial memory deficiency of memory-deficient mice. It can be seen from Figure 5B that the proportion of exploration duration in the HT-267, HT-177 and HT-178 treatment groups was significantly higher than that in the control group, indicating that compounds HT-267, HT-177 and HT-178 produced an improvement on spatial memory deficiency in memory-deficient mice, and the HT-267 treatment group had the best effect. Considering that compounds HT-267, HT-177 and HT-178 had a comparable therapeutic efficacy to CCK4, but fewer treatment times and fewer treatment days than CCK4, we believed that compounds HT-267, HT-177 and HT-178 had a greater therapeutic potential than CCK4.

This experiment showed that CCK4 and compounds HT-267, HT-177 and HT-178 produced an improvement on spatial memory deficiency in memory-deficient mice, and compounds HT-267, HT-177 and HT-178 had a greater therapeutic potential than CCK4.

### Example 7

The new object recognition experiment is a highly validated method of cognitive memory, designed by Ennaceur & Delacour (1988) based on the exploration characteristics of rodents to new environments, and this model evaluates the memory function of the tested animal based on the length of time that the animal spends in exploring familiar objects it has seen in the environment and new objects it has not seen. If the tested animal does not forget the familiar objects seen in the environment, it will spend more time exploring new objects that it has not seen; If the familiar objects that have been seen are forgotten, the animal should spend essentially the same amount of time exploring new objects in the environment and familiar objects it has seen. The experiment is usually divided into three stages: 1. Adaptation period: let the animal be familiar with the experimental environment, and no objects are placed in the experimental box; 2. Familiarization period: place two identical objects in the experimental box and let the mice explore freely for 10 minutes; 3. Test period: put a new object in the experimental box to replace one of the old objects, and the positions of the objects remain unchanged. The exploration of mice was recorded with video, and the time spent in exploring the old and new objects by mice was analyzed, and the proportion of time spent in exploring the new object by mice in the test period accounting for the total time for exploring the objects was calculated, that is, the recognition index of new objects.

Adult male tri-transgenic Alzheimer's mice (3xTg-AD) were housed under standard conditions with free access to food and water. Alzheimer's mice were shown to have memory deficiency in the new object recognition experiment and reduced ability to recognize new objects. We randomized Alzheimer's mice into two groups, one given intraperitoneal injection of the compound HT-267 solution and the other given intraperitoneal injection of the solvent of drug as a control. As shown in Figure 6B, the recognition index of new objects in the administration group was significantly higher than that of the control group, indicating that the administration improved the recognition memory of mice.

### Example 8

Adult memory-deficient mice were housed under standard conditions with free access to food and water. Our new object recognition experiment for testing memory-deficient mice mainly included the adaptation period, the familiarization period, and the test period. The experimental method was the same as Example 12.

We randomized memory-deficient mice into four groups, with one group given intraperitoneal injection of the solvent of drug as the control group, administration group 1 given intraperitoneal injection of the compound HT-267 solution, administration group 2 given intraperitoneal injection of the compound HT-177 solution, and administration group 3 given intraperitoneal injection of the compound HT-178 solution. After the adaptation and familiarization period training, we tested the recognition memory of several groups of mice in the test period experiment. Figure 7 compared the recognition index of new objects between four groups of mice during the test period, and it can be seen from Figure 7 that the recognition index of new objects in the three administration groups was significantly higher than that of the control group.

This experiment showed that compounds HT-267, HT-177 and HT-178 produced an improvement on recognition memory deficiency in memory-deficient mice.

### Example 9

Long-term potentiation (LTP) is considered by researchers to be one of the memory mechanisms. We used an in vitro electrophysiological electrical signal recording method to investigate whether compound HT-267 can induce long-term potentiation in the neocortex of senile mice.

We used an MED64 low-noise multi-electrode recording system to record the electrical signals of brain slices cultured in vitro. After stably recording excitatory postsynaptic field potentials for at least 15 minutes, we injected compound HT-267 to the brain slices for five minutes, and we found that the basal field potential was enhanced, and the enhancement lasted for more than one hour, as shown in Figure 8. This experiment showed that compound HT-267 could induce long-term potentiation in the cortex of senile mice.

### Example 10

An in vitro electrophysiological electrical signal recording method was used to investigate whether compound HT-267 can induce long-term potentiation in the neocortex of Alzheimer's mice. The MED64 low-noise multi-electrode recording system was used to record the electrical signals of brain slices cultured in vitro. After stably recording excitatory postsynaptic field potentials for at least 15 minutes, compound HT-267 was injected to the brain slices for five minutes, and the basal field potential was found to be enhanced, and the enhancement lasted for more than one hour, as shown in Figure 9. It was shown that compound HT-267 could induce long-term potentiation in the neocortex of Alzheimer's mice.

### Example 11

An in vitro electrophysiological electrical signal recording method was used to investigate whether compound HT-267, compound HT-177, and compound HT-178 can induce long-term potentiation in the neocortex of memory-deficient mice. The MED64 low-noise multi-electrode recording system was used to record the electrical signals of brain slices cultured in vitro. After stably recording excitatory postsynaptic field potentials for at least 15 minutes, compound HT-267 (administration group 1), compound HT-177 (administration group 2), and compound HT-178 (administration group 3) were respectively injected to brain slices for five minutes, and it was found that the basal field potential of each group of brain slices was enhanced, and the enhancement lasted for more than one hour, as shown in Figure 10. After normalization to the basal field potential before administration, the field potentials of three administration groups were significantly enhanced to 120-140%. This experiment showed that compound HT-267, compound HT-177, and compound HT-178 could induce long-term potentiation in the neocortex of memory-deficient mice.

The foregoing is only a preferred embodiment of the present invention, and it should be noted that for those skilled in the art, a number of improvements and modifications may also be made without departing from the principles of the present invention, and these improvements and modifications should also be regarded as within the scope of protection of the present invention.

## Claims

1. A polypeptide having a structure shown in Formula I or its stereoisomer, prodrug, pharmaceutically acceptable solvate or salt: 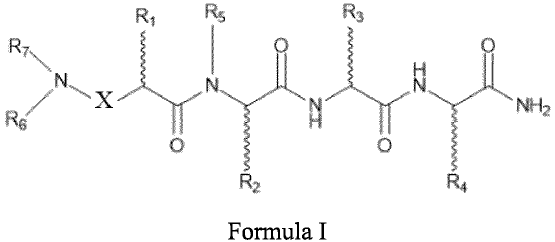
wherein: X is an amide bond or a single bond; 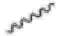 is 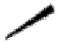 or 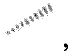 so that the amino acid at the corresponding position is
a D-type or L-type amino acid; R₁, R₂, R₃ and R₄ are independently selected from the group consisting of the following structures: 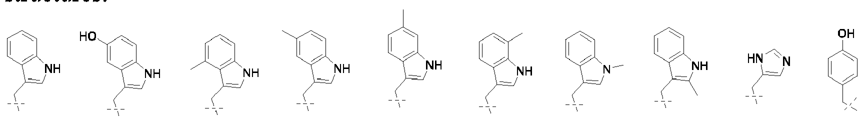 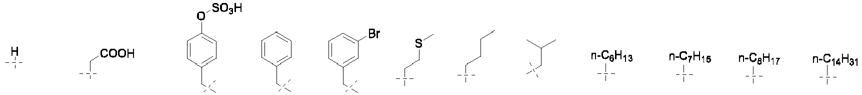 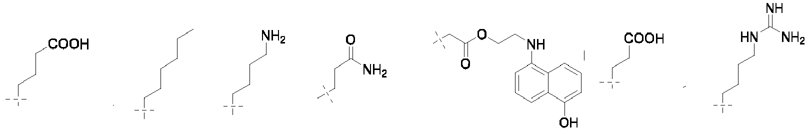 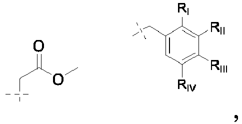 and R_{I}, R_{II}, R_{III} and R_{IV} are independently selected from the group consisting of halogen, nitro, C₁-C₄ alkyl, azido and cyano;
R₅ and R₆ are independently selected from the group consisting of H, and substituted or unsubstituted C₁-C₄ alkyl;
R₇ is selected from the group consisting of the following structures:
Biotin, AC, Fmoc, Cbz, PEG100, PEG200, PEG300, PEG400, PEG600, PEG800, PEG1000, PEG1500, PEG2000,

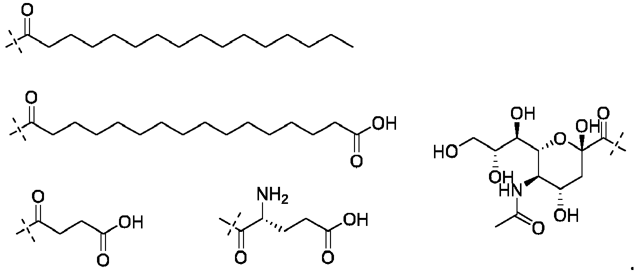

2. The polypeptide according to claim 1, wherein R₁ is selected from the group consisting of the following structures: 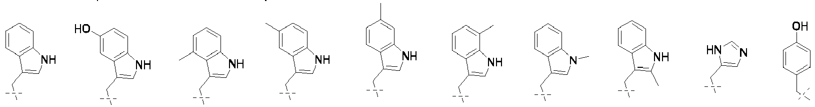 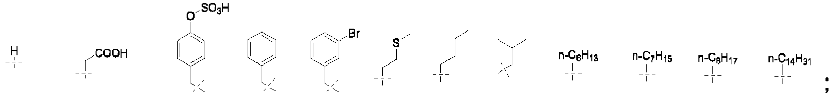
R₂ is selected from the group consisting of the following structures: 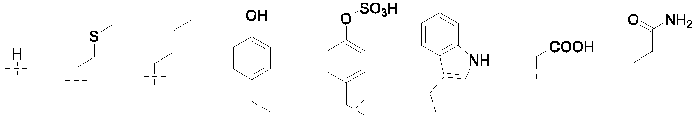 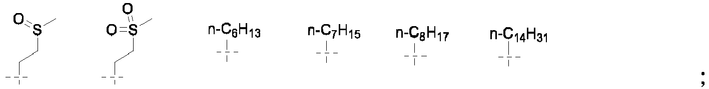
R₃ is selected from the group consisting of the following structures: 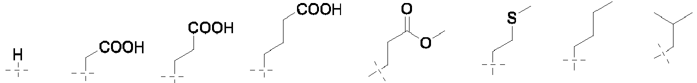 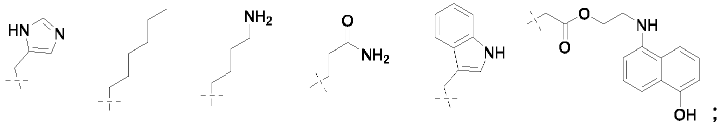
R₄ is selected from the group consisting of the following structures: 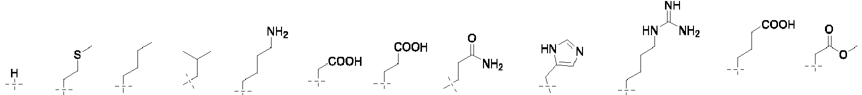 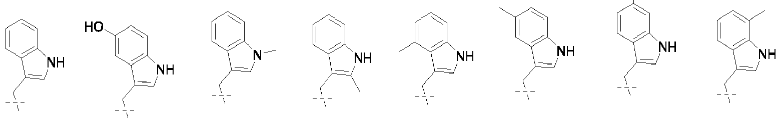 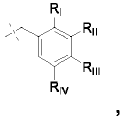
R_{I}, R_{II}, R_{III} and R_{IV} are independently selected from the group consisting of halogen, nitro, C₁-C₄ alkyl, azido and cyano.

3. The polypeptide according to claim 1, wherein the polypeptide has a structure shown in Formula II:

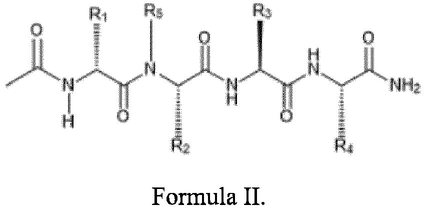

4. The polypeptide according to claim 3, wherein
R₁ is selected from the group consisting of the following structures: 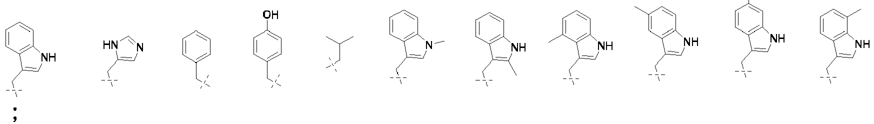
R₂ is selected from the group consisting of the following structures: 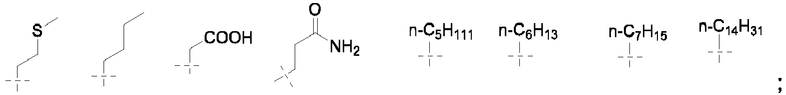
R₃ is selected from the group consisting of the following structures: 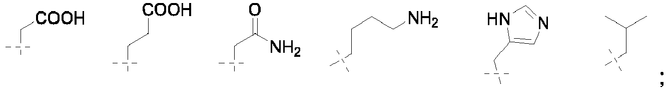
R₄ is selected from the group consisting of the following structures: 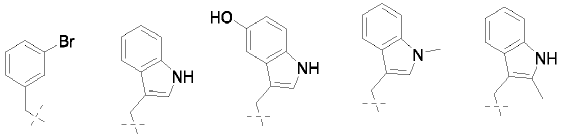 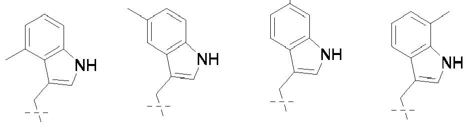
R₅ is selected from the group consisting of H and CH₃.

5. The polypeptide according to claim 1, wherein the polypeptide has a structure shown in Formula III or IV: 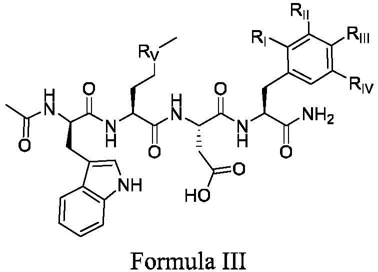 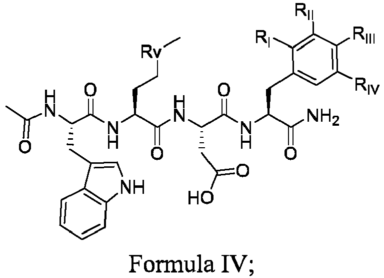 wherein R_{I}, R_{II}, R_{III} and R_{IV} are independently selected from the group consisting of halogen, nitro, C₁-C₄ alkyl, azido N₃ and cyano, and R_{V} is a carbon or sulfur atom.

6. The polypeptide according to claim 5, wherein R_{I}, R_{II}, R_{III} and R_{IV} are independently selected from the group consisting of H, F, Cl, Br, I, CN, N₃, Me and NO₂.

7. The polypeptide according to claim 1, wherein the polypeptide is any of compounds HT-1 to HT-292 in Table 1.

8. A pharmaceutical composition, wherein the composition comprises the polypeptide of any of claims 1-7, a pharmaceutically acceptable salt, stereoisomer or prodrug molecule thereof, and pharmaceutically acceptable excipients.

9. Use of the polypeptide of any of claims 1-7 or the pharmaceutical composition of claim 8 as a CCK receptor agonist or antagonist, or in the preparation of a medicament for treating or preventing a CCK receptor-related disease.

10. The use according to claim 9, wherein the CCK receptor-related disease includes at least one of amnesia, dementia, epilepsy, depression, obesity, gallbladder cancer, pancreatic cancer, and diseases of the digestive system.
